# EUROPEAN PATENT APPLICATION

(11) **EP 4 238 975 A1**
(43) Date of publication of application: **06.09.2023**
(21) Application number: 21885224.2
(22) Date of filing: 27.10.2021
(51) Int. Cl.: C07D 487/04, A61K 31/519, A61K 31/5377, A61P 25/28, A61P 35/00

(54) **SUBSTITUTED DIARYLAMINE COMPOUND, PHARMACEUTICAL COMPOSITION THEREOF, PREPARATION METHOD THEREFOR, AND USE THEREOF**

(30) Priority: 29.10.2020 CN 202011187029
(71) Applicant: Suzhou Yabao Pharmaceutical R&D Co., Ltd., Suzhou, Jiangsu 215123 (CN)
(72) Inventor: XIA, Yan, Suzhou, Jiangsu 215123 (CN); GUO, Zengshan, Suzhou, Jiangsu 215123 (CN); JIANG, Ziqing, Suzhou, Jiangsu 215123 (CN); WANG, Xizhi, Suzhou, Jiangsu 215123 (CN); ZHU, Taotao, Suzhou, Jiangsu 215123 (CN); WANG, Chuan, Suzhou, Jiangsu 215123 (CN); ZHUO, Lang, Suzhou, Jiangsu 215123 (CN)
(74) Representative: Gille Hrabal Partnerschaftsgesellschaft mbB Patentanwälte
(86) International application number: PCT/CN2021/126790
(87) International publication number: WO 2022/089497

(57) **Abstract**

A compound represented by formula (I), and a racemate, a stereoisomer, a tautomer, an isotopic label, an N-oxide, a hydrate, a solvate, a polymorph, a metabolite, a pharmaceutically acceptable salt, a pharmaceutically acceptable ester, or a prodrug compound thereof. This class of compounds has a good LRRK2 kinase regulation and inhibition and can be used for the treatment of an LRRK2 kinase activity-associated conditions and diseases, such as proliferative diseases, protein kinase-associated diseases, lysosomal diseases, Tau diseases, and diseases caused by decreased dopamine levels, etc., and is prepared by a simple method, and has good application prospects.

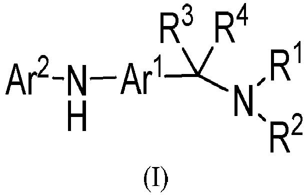

## Description

The present application claims priority to Chinese Patent Application No. 202011187029.7 filed with China National Intellectual Property Administration on Oct. 29, 2020 and entitled "SUBSTITUTED DIARYLAMINE COMPOUND, PHARMACEUTICAL COMPOSITION THEREOF, PREPARATION METHOD THEREFOR, AND USE THEREOF", which is incorporated herein by reference in its entirety.

### TECHNICAL FIELD

The present disclosure belongs to the technical field of pharmaceuticals, and particularly relates to a substituted diarylamine compound, a pharmaceutical composition thereof, a preparation method therefor, and use thereof.

### BACKGROUND

Parkinson's disease (PD) is a common neurodegenerative disease that affects 1-2% of the elderly. Genome-wide association studies (GWAS) have identified, in non-familial PD, 24 loci associated with 28 related genetic risk variations. Among them, mutations in LRRK2 (Park8) are also found in genetic forms, identifying common molecular pathway-driven pathogenesis of both familial and non-familial PD, the most common cause of related diseases (Simon-Sanchez J, Schulte C, Bras JM, Sharma M, Gibbs JR, et al., Genome-wide association study reveals genetic risk underlying Parkinson's disease. Nat Genet, 2009, 41, 1308-1312). The profile of PD pathogenic mutations in LRRK2 is mainly associated with the kinase (G2019S, I2020T) and the ROC-COR domain (R1441C/G/H, Y1699C), which means that these enzymatic activities are critical for disease development. Pathogenic mutations are rare, found in about 2% of the overall population. However, the most common mutation G2019S is found in up to 40% of patients in some ethnic groups, activating the kinase to increase its activity by two to three times (West AB, Moore DJ, Biskup S, Bugayenko A, Smith WW, et al., From The Cover: Parkinson's disease-associated mutations in leucine-rich repeat kinase 2 augment kinase activity. Proceedings of the National Academy of Sciences, 2005, 102, 16842-16847). In addition to pathogenic mutations, a common genetic variation in LRRK2 is a risk factor for sporadic PD (Tan, E. K. Identification of a common genetic risk variant (LRRK2 Gly2385Arg) in Parkinson's disease. Ann Acad Med Singapore. 2006, 35, 840-842).

In 2004, LRRK2 was identified as the gene responsible for PD inheritance, which was associated with the PARK8 locus and it was found to consist of 51 exons, resulting in one large (268kDa) protein. Subsequently, a number of primary structural variants of LRRK2 were identified, including dominant mutations separated from familial PD that also occur in sporadic PD, as well as polymorphisms at the LRRK2 loci that increase the lifetime risk of sporadic PD development.

LRRK2 is a multi-domain protein with two enzymatic functions at its core. The GTPase domain comprises the Ras of complex protein (ROC) which terminates in a spacer domain at the C-terminal of Roc domain (COR), followed by a kinase domain belonging to serine/threonine kinases. The enzymatic core is surrounded by protein-protein interaction domains, including the N-terminal leucine-rich repeat (LRR) domain of Armadillo, ankyrin, and LRRK2. The C-terminal of LRRK2 contains WD40 domain which is a region essential for protein folding and can control the functions and kinase activities of LRRK2 (Rudenko IN, Kaganovich A, Hauser DN, Beylina A, Chia R, et al., The G2385R variant of leucine-rich repeat kinase 2 associated with Parkinson's disease is a partial loss-of-function mutation. Biochemical Journal, 2012, 446, 99-111). Interestingly, the presence of the newly-described dominant pathogenic mutation in the enzymatic nucleus of LRRK2 indicates that the modification of LRRK2 activity greatly affects the development and progression of PD.

To date, nearly 40 single amino acid substitution mutations in the LRRK2 mutations have been associated with autosomal dominant PD. In European, the most common mutant form of LRRK2 comprises about 6% of familial PD cases and 3% of sporadic PD cases, including the amino acid substitution of Gly2019 with a Ser residue. Gly2019 is located within the conserved DYG-Mg²⁺ binding motif in subdomain-VII of the kinase domain. Recent reports have shown that this mutation enhances the autophosphorylation of LRRK2 and increases its ability to phosphorylate myelin basic protein 2-3 fold. Both of cytotoxicity and inclusion body formation are observed when G2019S-LRRK2 is overexpressed in cell lines and primary neuronal cultures, regardless of the presence of oxidative stress. These results, together with the genetic inactivation of LRRK2 kinase activity, show a protective effect on the toxic phenotype, which suggests that changes in LRRK2 kinase activity may be associated with neurotoxicity and pathogenic mechanisms of LRRK2-PD.

Studies have found that induced pluripotent stem cells (iPSCs) from LRRK2G 2019S patients with Parkinson's disease exhibit deficient neurite outgrowth and increased sensitivity to rotenone, which can be improved by genetic modification of the G2019S mutation or treatment of the cells with small molecule inhibitors of LRRK2 kinase activity (Reinhardt P, Schmid B, Burbulla Lena F, Schöndorf David C, Wagner L, et al., Genetic Correction of a LRRK2 Mutation in Human iPSCs Links Parkinsonian Neurodegeneration to ERK-Dependent Changes in Gene Expression. Cell Stem Cell, 2013, 12, 354-367). The increased mitochondrial damage associated with LRRK2G 2019S mutations in iSPC is also blocked by genetic correction of the G2019S mutation.

### SUMMARY

In order to solve the technical problems described above, the present disclosure provides a compound represented by formula (I), or a racemate, a stereoisomer, a tautomer, an isotopically labeled compound, an N-oxide, a hydrate, a solvate, a polymorph, a metabolite, a pharmaceutically acceptable salt, a pharmaceutically acceptable ester or a prodrug compound thereof: 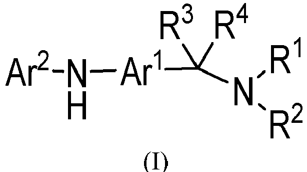 wherein,
R¹ and R², together with the atom attached thereto, form 3- to 20-membered heterocyclyl unsubstituted or optionally substituted with 1, 2 or more R^{b};
R³ and R⁴ are identical or different, and are each independently selected from H, CN, and the following groups unsubstituted or optionally substituted with 1, 2 or more R^{c}: C₁₋₄₀ alkyl, C₃₋₄₀ cycloalkyl, C₆₋₂₀ aryl, 5- to 20-membered heteroaryl, 3- to 20-membered heterocyclyl, C₁₋₄₀ alkyloxy, C₃₋₄₀ cycloalkyloxy, C₆₋₂₀ aryloxy, 5- to 20-membered heteroaryloxy, and 3- to 20-membered heterocyclyloxy; provided that R³ and R⁴ are not both H;
or, R³ and R⁴, together with the atom attached thereto, form the following group unsubstituted or optionally substituted with 1, 2 or more R^{d}: C₃₋₄₀ cycloalkyl or 3- to 20-membered heterocyclyl;
Ar¹ is selected from C₆₋₂₀ aryl and 5- to 20-membered heteroaryl substituted with 1, 2 or more R^{e};
Ar² is selected from C₆₋₂₀ aryl and 5- to 20-membered heteroaryl substituted with 1, 2 or more R^{f};
each of R^{b}, R^{c}, R^{d}, R^{e} and R^{f} is identical or different, and is independently selected from H, halogen, OH, CN, NO₂, oxo (=O), thio (=S), and the following groups unsubstituted or optionally substituted with 1, 2 or more R^{g}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₁₋₄₀ alkylthio, C₂₋₄₀ alkenylthio, C₂₋₄₀ alkynylthio, C₃₋₄₀ cycloalkylthio, C₃₋₄₀ cycloalkenylthio, C₃₋₄₀ cycloalkynylthio, C₆₋₂₀ arylthio, 5- to 20-membered heteroarylthio, 3- to 20-membered heterocyclylthio, NH₂, -C(O)R¹¹, -C(O)OR¹², -OC(O)R¹³, -S(O)₂R¹⁴, -S(O)₂OR¹⁵, and -OS(O)₂R¹⁶;
each R^{g} is identical or different, and is independently selected from H, halogen, OH, CN, NO₂, oxo (=O), thio (=S), C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₁₋₄₀ alkylthio, C₂₋₄₀ alkenylthio, C₂₋₄₀ alkynylthio, C₃₋₄₀ cycloalkylthio, C₃₋₄₀ cycloalkenylthio, C₃₋₄₀ cycloalkynylthio, C₆₋₂₀ arylthio, 5- to 20-membered heteroarylthio, 3- to 20-membered heterocyclylthio, NH₂, -C(O)C₁₋₄₀ alkyl, -C(O)NH₂, -C(O)NHC₁₋₄₀ alkyl, -C(O)-NH-OH, -COOC₁₋₄₀ alkyl, -COOH, -OC(O)C₁₋₄₀ alkyl, -OC(O)H, -S(O)₂C₁₋₄₀ alkyl, S(O)₂H, -S(O)₂OC₁₋₄₀ alkyl, and -OS(O)₂C₁₋₄₀ alkyl;
R¹¹, R¹², R¹³, R¹⁴, R¹⁵ and R¹⁶ are identical or different, and are each independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, and NH₂.

According to an embodiment of the present disclosure, R¹ and R², together with the atom attached thereto, form 3- to 10-membered heterocyclyl unsubstituted or optionally substituted with 1, 2 or more R^{b};
According to an embodiment of the present disclosure, R³ and R⁴ are identical or different, and are each independently selected from H, CN, and the following groups unsubstituted or optionally substituted with 1, 2 or more R^{c}: C₁₋₆ alkyl, C₃₋₈ cycloalkyl, C₁₋₆ alkyloxy, and C₃₋₈ cycloalkyloxy; provided that R³ and R⁴ are not both H;
or, R³ and R⁴, together with the atom attached thereto, form the following group unsubstituted or optionally substituted with 1, 2 or more R^{d}: C₃₋₈ cycloalkyl or 3- to 8-membered heterocyclyl.

According to an embodiment of the present disclosure, Ar¹ is selected from C₆₋₁₀ aryl and 5- to 10-membered heteroaryl substituted with 1, 2 or more R^{e}.

According to an embodiment of the present disclosure, Ar² is selected from C₆₋₁₀ aryl and 5- to 10-membered heteroaryl substituted with 1, 2 or more R^{f}.

According to an exemplary embodiment of the present disclosure, R¹ and R², together with the atom attached thereto, form the following group unsubstituted or optionally substituted with 1, 2 or more R^{b}:

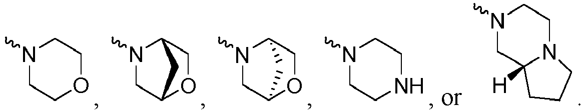

According to an exemplary embodiment of the present disclosure, R³ and R⁴ are identical or different, and are each independently selected from H, C₁₋₆ alkyl, and C₁₋₆ haloalkyl, such as H, methyl, and trifluoromethyl; provided that R³ and R⁴ are not both H;
or, R³ and R⁴, together with the atom attached thereto, form C₃₋₈ cycloalkyl, such as cyclopropyl.

According to an exemplary embodiment of the present disclosure, Ar¹ is selected from phenyl substituted with 1, 2 or more R^{e}.

According to an exemplary embodiment of the present disclosure, Ar² is selected from the following group substituted with 1, 2 or more R^{f}:

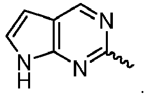

According to an embodiment of the present disclosure, the compound represented by formula (I) may have a structure represented by formula (II): 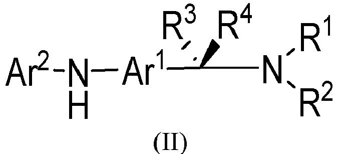 wherein R¹, R², R³, R⁴, Ar¹ and Ar² are independently defined as described above.

According to an embodiment of the present disclosure, the compound represented by formula (I) may be selected from the following compounds:

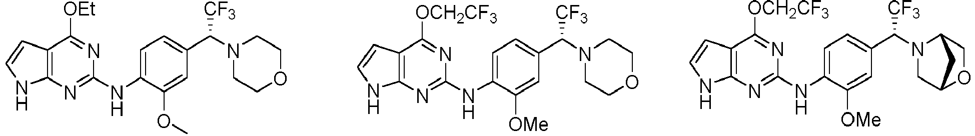

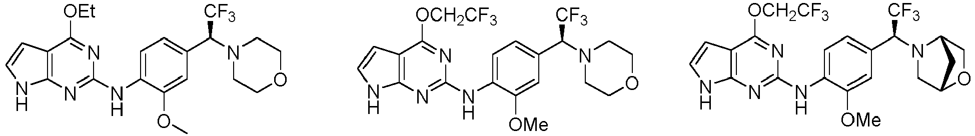

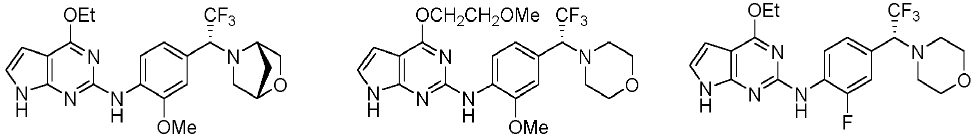

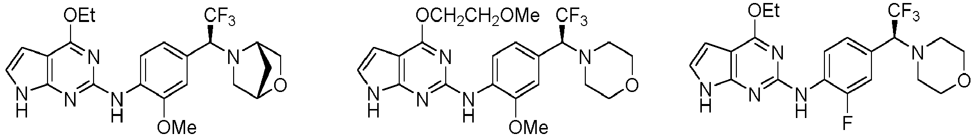

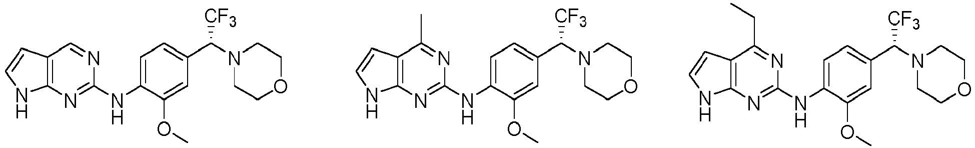

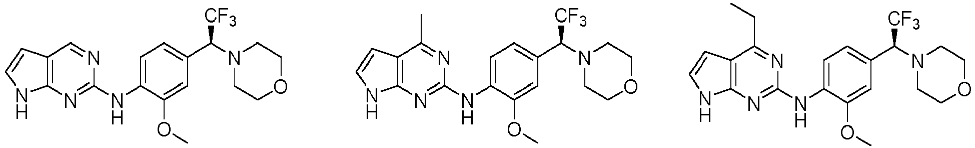

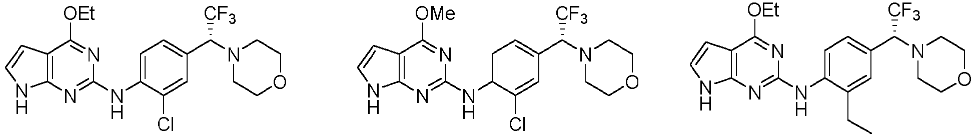

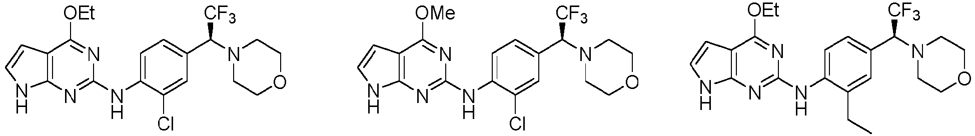

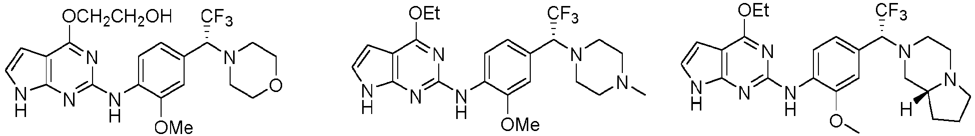

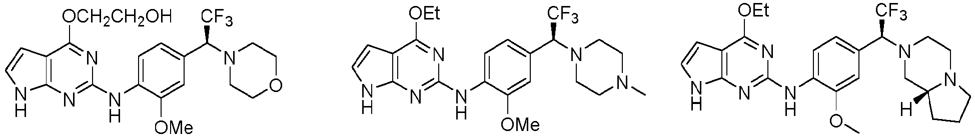

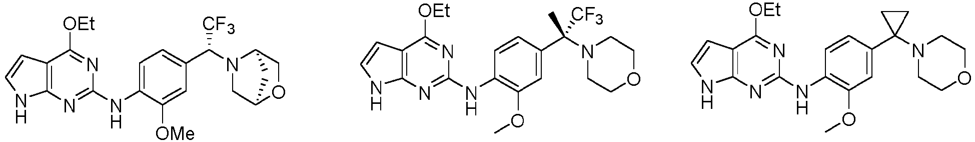

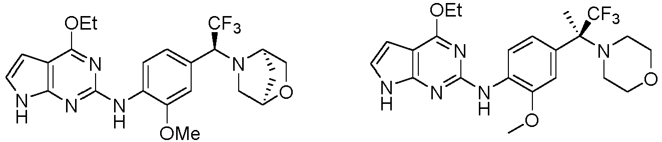

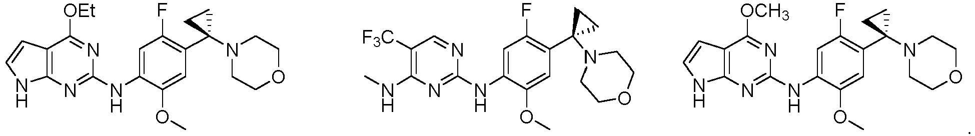

According to an embodiment of the present disclosure, the compounds covered by the general formula, for example the specific compounds specifically listed above and below in the context of the present disclosure, can independently have a chiral chemical structure, so that they can exist in the form of their various stereoisomers, mixtures of stereoisomers or racemates.

Thus, the compounds in the context of the present disclosure may be present as enantiomerically pure (+)-enantiomer or (-)-enantiomer (i.e., corresponding enantiomeric excess (ee) values > 99%), or as a mixture with (+)-enantiomeric excess or (-)-enantiomeric excess.

Those skilled in the art can clearly distinguish different enantiomers via the optical rotation [α]_{D} of the compounds of the present disclosure, whose stereoisomers (e.g., enantiomeric forms) can independently have a positive or negative optical rotation value. For example, different enantiomers can be clearly distinguished via the optical rotation [α]_{D} of the compounds of the present disclosure when the compounds are dissolved in an achiral solvent: one of the enantiomers has an optical rotation [α]_{D} greater than 0° (which may also be referred to as a (+)-compound), and the other enantiomer has an optical rotation [α]_{D} less than 0° (which may also be referred to as a (-)-compound).

As an example, an enantiomer of the following compound according to the present disclosure can has optical rotations greater than 0° and less than 0°, respectively:

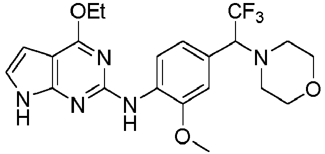

The enantiomers of the compound can be labeled compound 1 and compound 4, respectively, wherein:
compound 1 has an optical rotation < 0°, preferably < about -40°, and more preferably < about -50°, e.g., an optical rotation of about -52.5° in 1 mg/mL of ethanol solution;
compound 4 has an optical rotation >0°, preferably > about +20°, and more preferably > about +40°, for example an optical rotation of about +43.5° in 1 mg/mL of ethanol solution.

Alternatively, different enantiomers may be distinguished via differences in retention times of the mobile phases, for example via the separation by supercritical fluid chromatography (SFC) with a chiralpak-IC column (4.6 mm × 250 mm, 5 µm). When the column is eluted with CO₂-IPA (0.2% DEA), one enantiomer has a retention time t*_{R}* lower than the other. For example, t*_{R}* of compound 1 is < 2 min, such as about 1.94 min; t*_{R}* of compound 4 is > 2 min, such as about 2.33 min.

The present disclosure further provides a compound represented by formula (I-1): 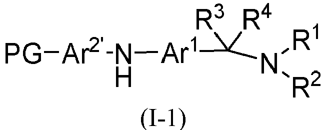
wherein PG is a protective group; Ar^{2'} is a substructure formed with an Ar² group losing one H;
R¹, R², R³, R⁴, Ar¹ and Ar² are independently defined as described above.

The present disclosure further provides use of the compound represented by formula (I-1) for the manufacturing of the compound represented by formula (I), or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester or the prodrug compound thereof.

The present disclosure further provides a preparation method for the compound represented by formula (I), 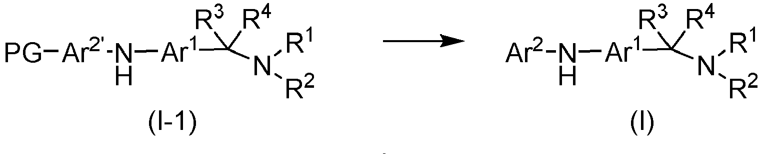 wherein PG, R¹, R², R³, R⁴, Ar¹, Ar² and Ar^{2'} are independently defined as described above.

According to an embodiment of the present disclosure, PG may be selected from amino protective groups. A suitable PG may be selected from C₁₋₄₀ alkyl, C₆₋₂₀ aryl C₁₋₄₀ alkyl, C₁₋₄₀ alkylsulfonyl, and C₁₋₄₀ alkylbenzenesulfonyl, such as 4-tosyl, tert-butyl, isopropyl, benzyl, *tert-*butoxycarbonyl (Boc), 2-biphenyl-2-propoxycarbonyl, benzyloxycarbonyl, fluorenylmethoxycarbonyl (Fmoc), and trifluoroacetyl.

According to an embodiment of the present disclosure, a compound of formula (I-1) is reacted after the protective group PG is removed off to obtain the compound of formula (I). The conditions for removing the protective group PG are those known to those skilled in the art.

The present disclosure further provides a preparation method for the compound represented by formula (I-1), which comprises reacting a compound of formula (I-2) with a compound of formula (I-3) to obtain the compound represented by formula (I-1); 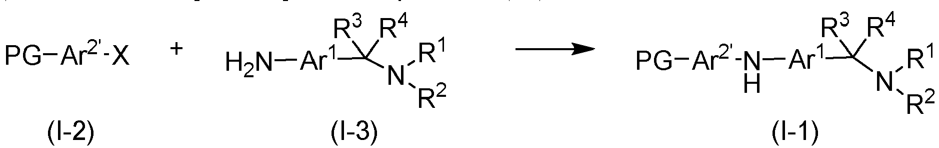 wherein PG, R¹, R², R³, R⁴, Ar¹, Ar² and Ar^{2'} are independently defined as described above.

According to an embodiment of the present disclosure, the preparation method may be carried out in the presence of a solvent such as an organic solvent. For example, the organic solvent may be selected from at least one of the following: alcohols such as methanol, ethanol, isopropanol and n-butanol; ethers such as ethyl propyl ether, n-butyl ether, anisole, phenetole, cyclohexylmethyl ether, dimethyl ether, diethyl ether, dimethyl glycol, diphenyl ether, dipropyl ether, diisopropyl ether, di-n-butyl ether, diisobutyl ether, diisoamyl ether, dimethoxyethane, isopropyl ethyl ether, methyl tert-butyl ether, tetrahydrofuran, methyltetrahydrofuran, dioxane, dichlorodiethyl ether, and polyethers of ethylene oxide and/or propylene oxide; aliphatic, cycloaliphatic or aromatic hydrocarbons such as pentane, hexane, heptane, octane, nonane, and those that may be substituted with fluorine and chlorine atoms, such as methylene chloride, dichloromethane, trichloromethane, tetrachloromethane, fluorobenzene, chlorobenzene or dichlorobenzene; cyclohexane, methylcyclohexane, petroleum ether, octane, benzene, toluene, chlorobenzene, bromobenzene, and xylene; and esters such as methyl acetate, ethyl acetate, butyl acetate, isobutyl acetate and dimethyl carbonate, dibutyl carbonate or ethylene carbonate.

According to an embodiment of the present disclosure, the preparation method may be performed in the presence of a catalyst. The catalyst may be a Pd catalyst, which may be selected from at least one of Pd₂(dba)₃, Pd(dba)₂, Pd(OAc)₂, Pd[(PPh)₃]₄, Pd[(PPh)₃]₂Cl₂, and Pd₂(dppf)Cl₂. The preparation method may further comprise adding a ligand, wherein the ligand may be an organic phosphine ligand, and the organic phosphine ligand may be selected from at least one of 2-dicyclohexylphosphino-2',6'-diisopropoxybiphenyl (RuPhos), 2-dicyclohexylphosphino-2'-(*N,N-*dimethylamine)-biphenyl (DavePhos), 2,2'-bis-diphenylphosphinoalkyl-[1,1']binaphthyl (BINAP) in a racemic or R- or S-configuration, 1,1-bis(diphenylphosphino)ferrocene (DPPF), and bis-(2-diphenylphosphinophenyl)ether (DPEPhos).

According to an embodiment of the present disclosure, the preparation method may be performed under the action of a base, wherein the base may be an inorganic base selected from at least one of sodium carbonate, potassium carbonate, potassium bicarbonate, sodium bicarbonate, and cesium carbonate.

The present disclosure further provides a pharmaceutical composition comprising a therapeutically effective amount of at least one of the compound represented by formula (I), and the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, and the prodrug compound thereof.

According to an embodiment of the present disclosure, the pharmaceutical composition further comprises one or more pharmaceutically acceptable adjuvants.

According to an embodiment of the present disclosure, the pharmaceutical composition may further comprise one or more additional therapeutic agents.

The present disclosure further provides a method for modulating LRRK kinase, particularly LRRK2 kinase activity, which comprises administering to a patient a prophylactically or therapeutically effective amount of at least one of a compound represented by formula (I), or a racemate, a stereoisomer, a tautomer, an isotopically labeled compound, an N-oxide, a hydrate, a solvate, a polymorph, a metabolite, a pharmaceutically acceptable salt, a pharmaceutically acceptable ester, and a prodrug compound thereof.

According to an embodiment of the present disclosure, LRRK kinase is preferably LRRK2 kinase, or a mutant or an isoform thereof, or a combination thereof.

The present disclosure further provides at least one of a compound represented by formula (I), and a racemate, a stereoisomer, a tautomer, an isotopically labeled compound, an N-oxide, a hydrate, a solvate, a polymorph, a metabolite, a pharmaceutically acceptable salt, a pharmaceutically acceptable ester and a prodrug compound thereof for use in diseases or disorders associated with LRRK kinase, particularly LRRK2 kinase activity.

According to an embodiment of the present disclosure, LRRK kinase is preferably LRRK2 kinase, or a mutant or an isoform thereof, or a combination thereof.

The diseases or disorders associated with LRRK kinase, particularly LRRK2 kinase activity, include: neurodegenerative diseases, proliferative diseases, diseases associated with protein kinases, lysosomal diseases, Tau disease and diseases caused by decreased dopamine levels, such as cancer (e.g., breast cancer), Parkinson's disease, Parkinson's disease (PD) associated with GBA mutations, other α-synucleinopathies, tau protein disease, cognitive impairment or cognitive disorder, Alzheimer's disease, Gaucher disease, Niemann-Pick type C (NPC), argyrophilic grain disease, Pick's disease, corticobasal degeneration, progressive supranuclear palsy, dementia, frontotemporal dementia with parkinsonism linked to chromosome 17 (FTDP-17), craniocerebral injury, stroke, epilepsy, withdrawal symptoms/relapse associated with drug addiction, inflammatory bowel diseases (e.g., Crohn's disease, and ulcerative colitis), leprosy, immune system diseases, and the like. Preferably, the diseases and disorders include those associated with the brain, in particular with blood supply to the brain or nerves, such as cognitive impairment or cognitive disorder, Alzheimer's disease, dementia, frontotemporal dementia with parkinsonism linked to chromosome 17 (FTDP-17), craniocerebral injury, stroke, and epilepsy.

In some embodiments, the patient is a human.

The present disclosure further provides a method for treating or preventing a disease or disorder associated with LRRK kinase, particularly LRRK2 kinase activity, which comprises administering to a patient at least one of a compound represented by formula (I), and a racemate, a stereoisomer, a tautomer, an isotopically labeled compound, an N-oxide, a hydrate, a solvate, a polymorph, a metabolite, a pharmaceutically acceptable salt, a pharmaceutically acceptable ester and a prodrug compound thereof.

The present disclosure further provides use of at least one of a compound represented by formula (I), and a racemate, a stereoisomer, a tautomer, an isotopically labeled compound, an N-oxide, a hydrate, a solvate, a polymorph, a metabolite, a pharmaceutically acceptable salt, a pharmaceutically acceptable ester and a prodrug compound thereof for the manufacturing of a medicament.

The medicament may be used for diseases or disorders associated with LRRK kinase, particularly LRRK2 kinase activity.

When used as a medicament, the compound of the present disclosure can be administered in the form of a pharmaceutical composition. Those compositions may be prepared in a manner well known in the pharmaceutical arts and may be administered by a variety of routes depending on whether local or systemic treatment is desired and the area to be treated. The pharmaceutical composition can be administered topically (e.g., by transdermal, dermal, ocular and mucosal routes, including intranasal, vaginal and rectal delivery routes), pulmonarily (e.g., by inhalation or insufflation of powders or aerosols, including by nebulizer; by intratracheal and intranasal routes), orally or parenterally. The parenteral administration includes intravenous, intra-arterial, subcutaneous, intraperitoneal or intramuscular injection or infusion; or intracranial (e.g., intrathecal or intracerebroventricular) administration. The pharmaceutical composition may be administered parenterally in a single bolus form, or may be administered by, for example, continuous infusion pump. The pharmaceutical composition and formulation administered topically may include a transdermal patch, an ointment, a lotion, a cream, a gel, a drop, a suppository, a spray, a liquid, a powder formulation, and a powder. Conventional pharmaceutical carriers, water, powders or oily bases, thickeners and the like may be necessary or desirable.

In preparing the composition of the present disclosure, the active ingredient is typically mixed with an excipient, diluted with an excipient or enclosed within such a carrier as capsules, sachets, paper or other container forms. When used as a diluent, the excipient may be a solid, semi-solid or liquid substance that serves as a vehicle, a carrier or a medium for the active ingredient. Thus, the composition may be in the following forms: tablets, pills, powders, lozenges, sachets, cachets, elixirs, suspensions, emulsions, solution formulations, syrups, aerosols (solid or dissolved in a liquid vehicle); ointments, soft and hard gelatin capsules, suppositories, sterile solutions for injection and sterile packaged powders containing, for example, up to 10% by weight of active compound.

Examples of suitable excipients can include lactose, glucose, sucrose, sorbitol, mannitol, starch, acacia, calcium phosphate, alginate, tragacanth, gelatin, calcium silicate, microcrystalline cellulose, polyvinylpyrrolidone, cellulose, water, syrup and methylcellulose. The formulation may further contain lubricants such as talc, sodium stearate, magnesium stearate, sodium oleate, sodium benzoate, sodium acetate, sodium chloride and mineral oil; wetting agents; emulsifiers and suspending agents; preservatives such as methyl benzoate and hydroxypropyl benzoate; and sweetening agents and flavoring agents. The composition of the present disclosure may be formulated by the methods known in the art so as to provide immediate, sustained or delayed release of the active ingredient after administration to the patient.

The composition may be formulated in unit dosage form. Each dose contains about 5-1000 mg, more typically about 100-500 mg, of the active ingredient. The term "unit dosage form" refers to physically discrete single dosage units suitable for use in human patients and other mammals, each unit containing a predetermined amount of active substance mixed with a suitable pharmaceutical excipient that can produce the desired therapeutic effect by calculation.

The active compound may be effective in a wide range of doses and is generally administered in a pharmaceutically effective amount. However, it should be understood that the amount of the compound actually administered is usually determined by a physician, in the light of the relevant circumstances, including the disorder to be treated, the selected route of administration, the compound actually administered; the age, weight and response of an individual patient; the severity of patient's symptoms and the like.

In preparing solid compositions such as tablets, the main active ingredient is mixed with pharmaceutical excipients to form a solid preformulation composition of a homogeneous mixture comprising the compound of the present disclosure. Where referring to these preformulation compositions as homogeneous, it is meant that the active ingredient is generally distributed evenly throughout the composition such that the composition may be readily divided into equally effective unit dosage forms such as tablets, pills and capsules. The solid preformulation is then divided into unit dosage forms of the type described above containing, for example, about 0.1-1000 mg of the active ingredient of the present disclosure.

The tablets or pills of the present disclosure may be coated or compounded to obtain a dosage form affording the advantage of long-acting effect. For example, the tablets or pills contain an inner dosage component and an outer dosage component, the latter being in the form of an envelope of the former. The two components may be separated by an enteric coating layer which serves to prevent the disintegration in the stomach to allow the inner component to pass through the duodenum entirely or to be delayed in release. A variety of substances may be used for such enteric coating layers or coating agents. Such substances include various polymeric acids and mixtures of polymeric acids and such substances as shellac, cetyl alcohol and cellulose acetate.

Liquid forms for oral administration or injection administration in which the compound and composition of the present disclosure may be incorporated include aqueous solutions, suitable flavoring syrups, aqueous or oil suspensions; and emulsions flavored with edible oils such as cottonseed oil, sesame oil, coconut oil or peanut oil; as well as elixirs and similar pharmaceutical vehicles.

Compositions for inhalation or insufflation include solution formulations and suspensions in pharmaceutically acceptable, aqueous or organic solvents, or mixtures thereof, and powders. The liquid or solid compositions may contain suitable pharmaceutically acceptable excipients as described above. In certain embodiments, the composition is administered by the oral or intranasal or respiratory route for local or systemic effect. The composition may be nebulized using an inert gas. The nebulized solution may be inhaled directly from the nebulizing device or the nebulizing device may be attached to a face mask, tent or an intermittent positive pressure ventilator. The solution, suspension or powder compositions may be administered orally, or nasally by means of a device which delivers the formulation in a suitable manner.

The amount of the compound or composition administered to a patient is not fixed and depends on the drug administered, the purpose of the administration such as prevention or treatment; the condition of the patient, the route of administration, etc. In therapeutic applications, the composition may be administered to a patient suffering from a disease in an amount sufficient to cure or at least partially arrest the symptoms of the disease and its complications. The effective dosage may depend on the disease state being treated and the determination of the attending clinician, the determination depending on factors such as the severity of the disease, the age, weight and general condition of the patient, and the like.

The composition administered to the patient may be in the form of the pharmaceutical composition as described above. These compositions may be sterilized by conventional sterilization techniques or by filter sterilization. The aqueous solutions may be packaged for use as is, or lyophilized, and the lyophilized formulation is mixed with a sterile aqueous carrier prior to administration. The pH of the compound formulation is usually 3-11, more preferably 5-9, and most preferably 7-8. It should be understood that the use of certain excipients, carriers or stabilizers as described above may result in the formation of a pharmaceutical salt.

The therapeutic dosage of the compound of the present disclosure can be determined, for example, according to: the specific use of the treatment, the route of administration of the compound, the health and condition of the patient, and the judgment of the prescriber. The proportion or concentration of the compound of the present disclosure in the pharmaceutical composition may vary depending on a variety of factors including the dosage, chemical properties (e.g., hydrophobicity), and the route of administration. For example, the compound of the present disclosure may be provided for parenteral administration by a physiological buffered aqueous solution containing about 0.1-10% w/v of the compound. Certain typical dosage ranges from about 1 µg/kg body weight/day to about 1 g/kg body weight/day. In certain embodiments, the dosage ranges from about 0.01 mg/kg body weight/day to about 100 mg/kg body weight/day. The dosage is likely to depend on such variables as the type and degree of progression of the disease or disorder, the general health condition of the particular patient, the relative biological potency of the compound selected, the excipient formulation and its route of administration. Effective dosage can be extrapolated from dose-response curves derived from *in vitro* or animal model test systems.

The present disclosure provides use of the compound represented by formula (I) in an analytical assay to identify the capacity of the compound to inhibit one or more kinases, preferably LRRK, and more preferably LRRK2.

Preferably, the analytical assay is a competitive binding assay.

More preferably, the competitive binding assay comprises contacting the compound of the present disclosure with a kinase and detecting any change in the interaction between the compound according to the present disclosure and the kinase.

Another aspect of the present disclosure provides a method for detecting the binding of a compound of the present disclosure to a kinase, the method comprising the following steps:
(i) contacting a compound of the present disclosure with a kinase in the presence of the kinase and a known substrate; and
(ii) detecting any change in an interaction between the kinase and the known substrate.

### Advantageous effects

The compound provided in the present disclosure has good LRRK2 kinase regulation/inhibition effect and more excellent blood-brain barrier permeability, and thus can be used for treating disorders and diseases associated with LRRK2 kinase activity, particularly disorders and diseases associated with the blood-brain barrier permeability, and can be used for preparing a medicament for the disorders and diseases. The compound of the present disclosure has simple preparation method and good application prospect.

### Definitions and Description

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should be construed as being within the scope of the present specification and/or the claims.

Unless otherwise stated, a numerical range set forth in the description and claims shall be construed as at least including each specific integer within the range. For example, the numerical range "1-40" shall be construed as at least including each integer value in the numerical range "1-10", i.e., 1, 2, 3, 4, 5, 6, 7, 8, 9 and 10, and each integer value in the numerical range "11-40", i.e., 11, 12, 13, 14, 15,..., 35, 36, 37, 38, 39 and 40. Moreover, when certain numerical ranges are defined as "numbers", it shall be construed as including both endpoints of the range, each integer within the range, and each decimal within the range. For example, "numbers of 0-10" shall be construed as including not only each of integers 0, 1, 2, 3, 4, 5, 6, 7, 8, 9 and 10, but also at least the sums of each integer and 0.1, 0.2, 0.3, 0.4, 0.5, 0.6, 0.7, 0.8 or 0.9.

It should be understood that when one, two or more are described herein, "more" shall mean an integer greater than 2, e.g., an integer greater than or equal to 3, e.g., 3, 4, 5, 6, 7, 8, 9 or 10.

The term "halogen" refers to fluorine, chlorine, bromine and iodine.

The term "C₁₋₄₀ alkyl" preferably refers to a linear or branched saturated monovalent hydrocarbyl group having 1-40 carbon atoms. For example, "C₁₋₁₀ alkyl" refers to a linear or branched alkyl group having 1, 2, 3, 4, 5, 6, 7, 8, 9 or 10 carbon atoms and "C₁₋₆ alkyl" refers to a linear or branched alkyl group having 1, 2, 3, 4, 5 or 6 carbon atoms. The alkyl is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof.

The term "C₂₋₄₀ alkenyl" preferably refers to a linear or branched monovalent hydrocarbyl containing one or more double bonds and having 2-40 carbon atoms, preferably "C₂₋₁₀ alkenyl". "C₂₋₁₀ alkenyl" preferably refers to a linear or branched monovalent hydrocarbyl containing one or more double bonds and having 2, 3, 4, 5, 6, 7, 8, 9 or 10 carbon atoms, for example, having 2, 3, 4, 5 or 6 carbon atoms (i.e., C₂₋₆ alkenyl) or having 2 or 3 carbon atoms (i.e., C₂₋₃ alkenyl). It should be understood that in the case that the alkenyl comprises more than one double bond, the double bonds can be separated from one another or conjugated. The alkenyl is, for example, vinyl, allyl, (E)-2-methylvinyl, (Z)-2-methylvinyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (*E*)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (*E*)-3-methylbut-1-enyl, (Z)-3-methylbut-1-enyl, (*E*)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylvinyl or 1-isopropylvinyl.

The term "C₂₋₄₀ alkynyl" refers to a linear or branched monovalent hydrocarbyl containing one or more triple bonds and having 2-40 carbon atoms, preferably "C₂₋₁₀ alkynyl". The term "C₂₋₁₀ alkynyl" preferably refers to a linear or branched monovalent hydrocarbyl containing one or more triple bonds and having 2, 3, 4, 5, 6, 7, 8, 9 or 10 carbon atoms, for example, having 2, 3, 4, 5 or 6 carbon atoms (i.e., "C₂₋₆ alkynyl") or having 2 or 3 carbon atoms ("C₂₋₃ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or 3,3-dimethylbut-1-ynyl. In particular, the alkynyl is ethynyl, prop-1-ynyl or prop-2-ynyl.

The term "C₃₋₄₀ cycloalkyl" refers to a saturated monovalent monocyclic or bicyclic (e.g., fused, bridged or spiro) hydrocarbon ring or tricyclic alkane having 3-40 carbon atoms, preferably "C₃₋₁₀ cycloalkyl". The term "C₃₋₁₀ cycloalkyl" refers to a saturated monovalent monocyclic or bicyclic (e.g., bridged or spiro) hydrocarbon ring or tricyclic alkane having 3, 4, 5, 6, 7, 8, 9 or 10 carbon atoms. The C₃₋₁₀ cycloalkyl may be monocyclic hydrocarbyl such as cyclopropyl, cyclobutyl, cyclopentyl, cyclohexyl, cycloheptyl, cyclooctyl, cyclononyl or cyclodecyl, or bicyclic hydrocarbyl such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonyl, 2,6-diazaspiro[3,4]octyl, or tricyclic hydrocarbyl such as adamantyl.

Unless otherwise defined, the term "3- to 20-membered heterocyclyl" refers to a saturated or unsaturated non-aromatic ring or ring system; for example, it is a 4-, 5-, 6- or 7-membered monocyclic ring system, a 7-, 8-, 9-, 10-, 11- or 12-membered bicyclic (e.g., fused, bridged or spiro) ring system or a 10-, 11-, 12-, 13-, 14- or 15-membered tricyclic ring system, and contains at least one, e.g., 1, 2, 3, 4, 5 or more heteroatoms selected from O, S and N, wherein N and S may also be optionally oxidized to various oxidized forms to form nitrogen oxides,-S(O)- or -S(O)₂-. Preferably, the heterocyclyl may be selected from "3- to 10-membered heterocyclyl". The term "3- to 10-membered heterocyclyl" refers to a saturated or unsaturated non-aromatic ring or ring system and contains at least one heteroatom selected from O, S and N. The heterocyclyl may be connected to the rest of the molecule through any of the carbon atoms or the nitrogen atom (if present). The heterocyclyl may include fused or bridged rings as well as spiro rings. In particular, the heterocyclyl may include, but is not limited to: 4-membered rings such as azetidinyl and oxetanyl; 5-membered rings such as tetrahydrofuranyl, dioxolyl, pyrrolidinyl, imidazolidinyl, pyrazolidinyl and pyrrolinyl; 6-membered rings such as tetrahydropyranyl, piperidyl, morpholinyl, dithianyl, thiomorpholinyl, piperazinyl and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[*c*]pyrrol-2(1*H*)-yl ring, or a 5,6-membered bicyclic ring such as a hexahydropyrrolo[1,2-*a*]pyrazin-2(1*H*)-yl ring. Heterocyclyl may be partially unsaturated, i.e., it may contain one or more double bonds, for example, but not limited to, dihydrofuranyl, dihydropyranyl, 2,5-dihydro-1H-pyrrolyl, 4H-[1,3,4]thiadiazinyl, 4,5-dihydrooxazolyl or 4H-[1,4]thiazinyl, or it may be benzo-fused, for example, but not limited to, dihydroisoquinolyl. When the 3- to 20-membered heterocyclyl is connected to another group to form the compound of the present disclosure, the group may be connected to the carbon atom on the 3- to 20-membered heterocyclyl, or may be connected to the heteroatom on the 3- to 20-membered heterocyclyl. For example, when the 3- to 20-membered heterocyclyl is selected from piperazinyl, the group may be connected to the nitrogen atom on the piperazinyl. Alternatively, when the 3- to 20-membered heterocyclyl is selected from piperidyl, the group may be connected to the nitrogen atom on the piperidyl ring or the carbon atom in the para position.

The term "C₆₋₂₀ aryl" preferably refers to an aromatic or partially aromatic monovalent monocyclic, bicyclic (e.g., fused, bridged or spiro) or tricyclic hydrocarbon ring having 6-20 carbon atoms, which may be a single aromatic ring or multiple aromatic rings fused together, preferably "C₆₋₁₄ aryl". The term "C₆₋₁₄ aryl" preferably refers to an aromatic or partially aromatic monovalent monocyclic, bicyclic or tricyclic hydrocarbon ring having 6, 7, 8, 9, 10, 11, 12, 13 or 14 carbon atoms ("C₆₋₁₄ aryl"), in particular a ring having 6 carbon atoms ("C₆ aryl"), such as phenyl or biphenyl, a ring having 9 carbon atoms ("C₉ aryl"), such as indanyl or indenyl, a ring having 10 carbon atoms ("C₁₀ aryl"), such as tetrahydronaphthyl, dihydronaphthyl or naphthyl, a ring having 13 carbon atoms ("C₁₃ aryl"), such as fluorenyl, or a ring having 14 carbon atoms ("C₁₄ aryl"), such as anthryl. When the C₆₋₂₀ aryl is substituted, it may be monosubstituted or polysubstituted. In addition, the substitution site is not limited, and may be, for example, ortho-substitution, para-substitution, or metasubstitution.

The term "5- to 20-membered heteroaryl" refers to a monovalent monocyclic, bicyclic (e.g., fused, bridged or spiro) or tricyclic aromatic ring system which has 5-20 ring atoms and contains 1-5 heteroatoms independently selected from N, O and S, such as "5- to 14-membered heteroaryl". The term "5- to 14-membered heteroaryl" refers to a monovalent monocyclic, bicyclic or tricyclic aromatic ring system that has 5, 6, 7, 8, 9, 10, 11, 12, 13 or 14 ring atoms, in particular 5, 6, 9 or 10 carbon atoms, contains 1-5, preferably 1-3 heteroatoms independently selected from N, O and S, and may be benzo-fused in each case. "Heteroaryl" also refers to a group in which a heteroaromatic ring is fused to one or more aryl, alicyclic or heterocyclyl rings, wherein the radical or site of attachment is on the heteroaromatic ring. Non-limiting examples of heteroaryl include 1-, 2-, 3-, 5-, 6-, 7- or 8-indolizinyl, 1-, 3-, 4-, 5-, 6- or 7-isoindolyl, 2-, 3-, 4-, 5-, 6- or 7-indolyl, 2-, 3-, 4-, 5-, 6- or 7-indazolyl, 2-, 4-, 5-, 6-, 7- or 8-purinyl, 1-, 2-, 3-, 4-, 6-, 7-, 8- or 9-quinolizinyl, 2-, 3-, 4-, 5-, 6-, 7-or 8-quinolyl, 1-, 3-, 4-, 5-, 6-, 7- or 8-isoquinolyl, 1-, 4-, 5-, 6-, 7- or 8-phthalazinyl, 2-, 3-, 4-, 5- or 6-naphthyridinyl, 2-, 3-, 5-, 6-, 7- or 8-quinazolinyl, 3-, 4-, 5-, 6-, 7- or 8-cinnolinyl, 2-, 4-, 6-or 7-pteridinyl, 1-, 2-, 3-, 4-, 5-, 6-, 7- or 8-4aH-carbazolyl, 1-, 2-, 3-, 4-, 5-, 6-, 7- or 8-carbazolylcarbazolyl, 1-, 3-, 4-, 5-, 6-, 7-, 8- or 9-carbolinyl, 1-, 2-, 3-, 4-, 6-, 7-, 8-, 9- or 10-phenanthridinyl, 1-, 2-, 3-, 4-, 5-, 6-, 7-, 8- or 9-acridinyl, 1-, 2-, 4-, 5-, 6-, 7-, 8- or 9-pyridinyl, 2-, 3-, 4-, 5-, 6-, 8-, 9- or 10-phenanthrolinyl, 1-, 2-, 3-, 4-, 6-, 7-, 8- or 9-phenazinyl, 1-, 2-, 3-, 4-, 6-, 7-, 8-, 9- or 10-phenothiazinyl, 1-, 2-, 3-, 4-, 6-, 7-, 8-, 9- or 10-phenazinyl, 2-, 3-, 4-, 5-, 6- or 1-, 3-, 4-, 5-, 6-, 7-, 8-, 9- or 10-benzisoquinolyl, 2-, 3-, 4- or thieno[2,3-b]furanyl, 2-, 3-, 5-, 6-, 7-, 8-, 9-, 10-or 11-7*H*-pyrazino[2,3-*c*]carbazolyl, 2-, 3-, 5-, 6- or 7-2*H*-furo[3,2-*b*]-pyranyl, 2-, 3-, 4-, 5-, 7- or 8-5H-pyrido[2,3-d]-o-oxazinyl, 1-, 3- or 5-1*H*-pyrazolo[4,3-*d*]-thiazolyl, 2-, 4- or 5-1*H*-imidazo[4,5-d]thiazolyl, 3-, 5- or 8-pyrazino[2,3-d]pyridazinyl, 2-, 3-, 5- or 6-imidazo[2,1-*b*]thiazolyl, 1-, 3-, 6-, 7-, 8- or 9-furo[3,4-c]cinnolinyl, 1-, 2-, 3-, 4-, 5-, 6-, 8-, 9-, 10- or 11-4*H*-pyrido[2,3-c]carbazolyl, 2-, 3-, 6- or 7-imidazo[1,2-b][1,2,4]triazinyl, 7-benzo[b]thienyl, 2-, 4-, 5-, 6- or 7-benzoxazolyl, 2-, 4-, 5-, 6- or 7-benzimidazolyl, 2-, 4-, 4-, 5-, 6- or 7-benzothiazolyl, 1-, 2-, 4-, 5-, 6-, 7-, 8- or 9-benzoxapinyl, 2-, 4-, 5-, 6-, 7- or 8-benzoazinyl, and 1-, 2-, 3-, 5-, 6-, 7-, 8-, 9-, 10- or 11-1*H-*pyrrolo[1,2-b][2]benzazapinyl. Typical fused heteroaryl groups include, but are not limited to, 2-, 3-, 4-, 5-, 6-, 7- or 8-quinolyl, 1-, 3-, 4-, 5-, 6-, 7- or 8-isoquinolyl, 2-, 3-, 4-, 5-, 6- or 7-indolyl, 2-, 3-, 4-, 5-, 6- or 7-benzo[b]thienyl, 2-, 4-, 5-, 6- or 7-benzoazolyl, 2-, 4-, 5-, 6- or 7-benzimidazolyl and 2-, 4-, 5-, 6- or 7-benzothiazolyl. When the 5- to 20-membered heteroaryl is connected to another group to form the compound of the present disclosure, the group may be connected to the carbon atom on the 5- to 20-membered heteroaryl ring, or may be connected to the heteroatom on the 5- to 20-membered heteroaryl ring. When the 5- to 20-membered heteroaryl is substituted, it may be monosubstituted or polysubstituted. In addition, the substitution site is not limited. For example, hydrogen connected to the carbon atom on the heteroaryl ring may be substituted, or hydrogen connected to the heteroatom on the heteroaryl ring may be substituted.

The term "spiro rings" refers to a ring system in which two rings share 1 ring-forming atom.

The term "fused rings" refers to a ring system in which two rings share 2 ring-forming atoms.

The term "bridged rings" refers to a ring system in which two rings share 3 or more ring-forming atoms.

Unless otherwise stated, the heterocyclyl, heteroaryl or heteroarylene includes all possible isomeric forms thereof, e.g., positional isomers thereof. Thus, for some illustrative non-limiting examples, forms that involving substitutions at or bonding to other groups at one, two or more of positions 1, 2, 3, 4, 5, 6, 7, 8, 9, 10, 11, 12 and the like (if present) are included, including pyridin-2-yl, pyridinylene-2-yl, pyridin-3-yl, pyridinylene-3-yl, pyridin-4-yl and pyridinylene-4-yl; thienyl or thienylene, including thien-2-yl, thien-2-ylene, thien-3-yl, and thien-3-ylene; pyrazol-1-yl, pyrazol-3-yl, pyrazol-4-yl and pyrazol-5-yl.

The term "oxo" means that the carbon atom, nitrogen atom or sulfur atom in the substituent is substituted with an oxy group formed after oxidation (=O).

Definitions of terms herein apply equally to groups containing the term unless otherwise stated. For example, the definition of C₁₋₆ alkyl also applies to C₁₋₆ alkyloxy, C₃₋₈ cycloalkyl-C₁₋₆alkyl-, etc.

It should be understood by those skilled in the art that the compound represented by formula (I) may be present in the form of various pharmaceutically acceptable salts. If these compounds have basic centers, they can form acid addition salts; if these compounds have acidic centers, they can form base addition salts; if these compounds contain both acidic centers (e.g., carboxyl) and basic centers (e.g., amino), they can also form internal salts.

The compound disclosed herein may exist in the form of a solvate (e.g., hydrate), and the compound disclosed herein contains a polar solvent as a structural element of the crystal lattice of the compound, particularly, for example, water, methanol or ethanol. The amount of polar solvent, especially water, can exist in a stoichiometric or non-stoichiometric ratio.

According to the molecular structure, the compounds disclosed herein may be chiral and may therefore exist in various enantiomeric forms. These compounds may therefore exist in racemic or optically active form. The compounds disclosed herein encompass isomers with each chiral carbon in R or S configuration, or mixtures and racemates thereof. The compounds disclosed herein or intermediates thereof may be separated into enantiomers by chemical or physical methods well known to those skilled in the art, or used in this form for synthesis. In the case of racemic amines, diastereoisomers are manufactured from mixtures by reaction with optically active resolving agents. Examples of suitable resolving agents are optically active acids such as R- or S-tartaric acid, diacetyltartaric acid, dibenzoyltartaric acid, mandelic acid, malic acid, lactic acid, suitable N-protected amino acids (e.g., N-benzoylproline or N-benzenesulfonylproline) or various optically active camphorsulfonic acids. Enantiomeric resolution by chromatography can be advantageously performed with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvent containing water or alcohol, for example, hexane/isopropanol/acetonitrile.

The corresponding stable isomers can be separated according to known methods, such as extraction, filtration or column chromatography.

The term "patient" refers to any animal including mammals, preferably mice, rats, other rodents, rabbits, dogs, cats, pigs, cattle, sheep, horses or primates, and most preferably humans.

The term "therapeutically effective amount" refers to the amount of the active compound or drug that causes a biological or medical response that researchers, veterinarians, physicians or other clinicians are looking for in tissues, systems, animals, individuals or humans, including one or more of the following effects: (1) disease prevention: for example, the prevention of a disease, disorder or condition in an individual who is susceptible to the disease, disorder or condition but has not yet experienced or exhibited the pathology or symptoms of the disease; (2) disease inhibition: for example, the inhibition of a disease, disorder or condition in an individual who is experiencing or exhibiting the pathology or symptoms of the disease, disorder or condition.(i.e., the prevention of the further development of the pathology and/or symptoms); and (3) disease alleviation: for example, the alleviation of a disease, disorder or condition in an individual who is experiencing or exhibiting the pathology or symptoms of the disease, disorder or condition (i.e., the reverse of the pathology and/or symptoms). The therapeutically effective amount can be estimated initially from cell culture assays, or the initial dose can be estimated from *in-vivo* data. Using these preliminary guidance, those of ordinary skill in the art can determine effective dosages for humans. In addition, the toxicity and the therapeutic efficacy of the compounds described herein can also be determined by standard pharmaceutical procedures in cell cultures or experimental animals, e.g., by the measurement of LD₅₀ and ED₅₀.

The term "disease or disorder associated with LRRK kinase activity" refers to a disease or disorder characterized by inappropriate such kinase activity or overactivity of a kinase, as defined herein. Inappropriate activity refers to (i) kinase expression in cells that generally do not express the kinase; (ii) increased kinase expression, resulting in undesired cell proliferation, differentiation and/or growth; or (iii) reduced kinase expression, resulting in an undesirable reduction in cell proliferation, differentiation and/or growth. The overactivity of a kinase refers to the amplification of a gene encoding a particular kinase or the generation of a kinase activity level that may be associated with a disorder of cell proliferation, differentiation and/or growth (i.e., as the level of kinase increases, the severity of one or more symptoms of the cellular disorder increases). The overactivity may also be the result of unrelated or constitutive activation of the ligand due to mutations such as deletion of a fragment of the kinase responsible for ligand binding.

Examples of "adjuvants" as described herein are found in "Handbook of Pharmaceutical Excipients, 2nd edition, 1994, edited by A Wade and PJ Weller".

The "carriers" or "diluents" described herein are well known in the pharmaceutical art and are described, for example, in Remington's Pharmaceutical Sciences, Mack Publishing Co. (A.R. Gennaro, edited in 1985).

The choice of pharmaceutical carriers, adjuvants or diluents can be selected with regard to the intended route of administration and standard pharmaceutical practice. The pharmaceutical composition may contain, or additionally contain, any suitable adhesives, lubricants, suspending agents, coating agents, solubilizers, buffers, flavoring agents, surfactants, thickening agents, preservatives (including antioxidants), and the like as carriers, adjuvants or diluents, as well as substances included to render the formulation isotonic with the blood of the recipient.

Pharmaceutical formulations suitable for oral administration with the carrier being a solid are most preferably in the form of unit dose formulations such as pills, capsules or tablets each containing a predetermined amount of the active compound. Tablets may be prepared by compression or molding, optionally together with one or more additional ingredients. Compressed tablets may be prepared by compressing in a suitable machine the active compound in a free-flowing form (such as powders or granules), optionally mixed with an adhesive, a lubricant, an inert diluent, a lubricating substance, a surfactant or a dispersing agent. Molded tablets may be prepared by molding the active compound with an inert liquid diluent. The tablets may optionally be coated and if not, symbols may optionally be printed thereon. Capsules may be prepared by filling capsule shells with the active compound alone or in admixture with one or more additional ingredients and then sealing in a conventional manner. Cachets are similar to capsules in that the active compound is enclosed in rice paper film along with any additional ingredients. The active compound may also be formulated into dispersible granules, so as to, for example, suspend it in water or spread it on food before administration. The granules may be packaged in, for example, a sachet. The carriers may be liquid formulations suitable for oral administration, and may be presented in the form of a solution or suspension in an aqueous or non-aqueous liquid, or in the form of an oil-in-water liquid emulsion.

The term "pharmaceutically acceptable salt" includes suitable acid addition salts or base salts thereof. Suitable pharmaceutical salts are described in J Pharm Sci., 66, 199, 1977, Berge et al. For example, salts are formed with strong inorganic acids, such as mineral acids, for example hydrohalic acids (e.g. hydrochloric acid, hydrobromic acid, and hydroiodic acid), sulfuric acid, phosphoric acid sulfate, hydrosulfate, hemisulfate, thiocyanate, persulfate, and sulfonic acid; with strong organic carboxylic acids, such as unsubstituted or substituted (e.g., halogen-substituted) alkanecarboxylic acids of 1 to 4 carbon atoms, such as acetic acid; with saturated or unsaturated dicarboxylic acids, such as oxalic acid, malonic acid, succinic acid, maleic acid, fumaric acid, phthalic acid or tetraphthalic acid; with hydroxycarboxylic acids, such as ascorbic acid, glycolic acid, lactic acid, malic acid, tartaric acid or citric acid; with amino acids, such as aspartic acid or glutamic acid; with benzoic acid; or with organic sulfonic acids, such as unsubstituted or substituted (e.g., halogen-substituted) (C₁-C₄)-alkyl- or aryl-sulfonic acid, such as methane-, orp-toluenesulfonic acid.

Preferred salts include, for example, acetate, trifluoroacetate, lactate, gluconate, citrate, tartrate, maleate, malate, pantothenate, adipate, alginate, aspartate, benzoate, butyrate, digluconate, cyclovalerate, glucoheptonate, glycerophosphate, oxalate, heptanoate, hexanoate, fumarate, nicotinate, palmitate, pectate, 3-phenylpropionate, picrate, pivalate, propionate, tartrate, lactobionate, pivolate, camphorate, undecanoate, and succinate; organic sulfonic acids such as methanesulfonate, ethanesulfonate, 2-hydroxyethanesulfonate, camphorsulfonate, 2-naphthalenesulfonate, benzenesulfonate, p-chlorobenzenesulfonate, and p-toluenesulfonate; and inorganic acids such as hydrochloric acid, hydrobromic acid, hydroiodic acid, sulfuric acid, hydrogen sulfate, hemisulfuric acid, thiocyanic acid, persulfuric acid, phosphoric acid, and sulfonic acid.

The term "pharmaceutically acceptable ester" refers to the use of an organic acid or alcohol/hydroxide to form an ester with a functional group that may be esterified in the structure of the compound of the present disclosure. Organic acids include carboxylic acids, such as unsubstituted or substituted (e.g., halogen-substituted) alkanecarboxylic acids of 1 to 12 carbon atoms, such as acetic acid; with saturated or unsaturated dicarboxylic acids, such as oxalic acid, malonic acid, succinic acid, maleic acid, fumaric acid, phthalic acid or tetraphthalic acid; with hydroxycarboxylic acids, such as ascorbic acid, glycolic acid, lactic acid, malic acid, tartaric acid or citric acid; with amino acids, such as aspartic acid or glutamic acid; with benzoic acid; or with organic sulfonic acids, such as unsubstituted or substituted (e.g., halogen-substituted) (C₁-C₄)-alkyl- or aryl-sulfonic acid, such as methane-, or *p*-toluenesulfonic acid. Suitable hydroxides include inorganic hydroxides such as sodium hydroxide, potassium hydroxide, calcium hydroxide, and aluminum hydroxide. Alcohols include alkanols of 1 to 12 carbon atoms that may be unsubstituted or substituted (e.g., with halogen).

The term "isotopically labeled compound" means that at least one atom in the compound of the present disclosure is replaced with an isotope. Examples of the isotopes include isotopes of hydrogen, carbon, nitrogen, oxygen, phosphorus, sulfur, fluorine, and chlorine, such as the corresponding ²H, ³H, ¹³C, ¹⁴C, ¹⁵N, ¹⁷O, ¹⁸O, ³¹P, ³²P, ³⁵S, ¹⁸F, and ³⁶Cl. Substitution with isotopes such as deuterium (i.e., ²H) may afford certain therapeutic advantages (i.e., increased *in-vivo* half-life or reduced dose requirements) resulting from greater metabolic stability and hence may be preferred in some circumstances. For example, the present disclosure comprises compounds of general formula (I) where any hydrogen atom is replaced with a deuterium atom.

The term "prodrug compound" refers to a covalently-bonded compound that releases the active parent drug according to general formula (I) *in vivo.* Such the prodrug is generally the compound of the present disclosure where one or more appropriate groups have been modified such that, upon administration to a human or mammalian subject, the modification may be reversed. The reversion is typically performed by an enzyme naturally present in such subjects, although it is possible to administer a second medicament with such the prodrug in order to allow for reversion *in vivo.* Examples of such modifications include pharmaceutically acceptable esters as described above, wherein such reversion may be performed via esterases and the like.

The term "polymorph" refers to the compound of the present disclosure in various forms of crystallines, polymorphs, and hydrates. It is well established in the pharmaceutical industry that chemical compounds can be isolated in any of these forms by methods of purification and/or separation of the solvents used in the synthetic manufacturing of such compounds.

The term "administration" means that the pharmaceutical composition of the present disclosure may be suitable for rectal, intranasal, intrabronchial, topical (including buccal and sublingual), vaginal or parenteral (including subcutaneous, intramuscular, intravenous, intraarterial, and intradermal), intraperitoneal or intrathecal administration. Preferably, the formulation is a formulation for oral administration. The formulations may conveniently be presented in unit dosage form, i.e., in the form of discrete parts containing a unit dose or a plurality of units or subunits of a unit dose. As an example, the formulation may be in the form of tablets and sustained release capsules, and may be prepared by any of the methods well known in the pharmaceutical art.

The formulation for oral administration of the present disclosure may be presented as: discrete units such as capsules, gels, drops, cachets, pills, or tablets each containing a predetermined amount of the active agent; as powders or granules; as a solution, emulsion or suspension of the active agent in an aqueous or non-aqueous liquid; or as an oil-in-water liquid emulsion or a water-in-oil liquid emulsion; or as a bolus preparation, and the like. Preferably, these compositions contain 1-250 mg and more preferably 10-100 mg of the active ingredient per dose.

For compositions for oral administration (e.g., tablets and capsules), solvents and/or conventional adjuvants are also included, such as adhesives, for example, syrup, acacia, gelatin, sorbitol, tragacanth, polyvinylpyrrolidone (povidone), methylcellulose, ethylcellulose, sodium carboxymethylcellulose, hydroxypropylmethylcellulose, sucrose, and starch; fillers and carriers, for example, corn starch, gelatin, lactose, sucrose, microcrystalline cellulose, kaolin, mannitol, dicalcium phosphate, sodium chloride, and alginic acid; and lubricants, for example, magnesium stearate, sodium stearate and other metal stearates, glyceryl stearate, stearic acid, silicone fluids, talc waxes, oils, and colloidal silica. Flavoring agents such as peppermint, wintergreen oil, cherry flavoring, and the like can also be used. It may be desirable to add a colorant to make the dosage form easily identifiable. Tablets may also be coated by methods well known in the art.

Other formulations suitable for oral administration include lozenges containing the active agent in a flavored matrix, usually sucrose and acacia or tragacanth; pastilles containing the active agent in an inert matrix such as gelatin and glycerin, or sucrose and acacia; and mouthwashes containing the active agent in a suitable liquid carrier.

Other forms of administration include solutions or emulsions prepared from sterile or sterilizable solutions for intravenous, intraarterial, intrathecal, subcutaneous, intradermal, intraperitoneal, or intramuscular injection. Injectable forms generally contain 10-1000 mg, preferably 10-250 mg, of the active ingredient per dose.

The forms of administration may also be a combination administration, i.e., one or more of the compounds of the present disclosure are administered in combination with one or more of other active agents. In such cases, the compound of the present disclosure may be administered with one or more of other active agents consecutively, simultaneously or sequentially.

### Analytical assay

Another aspect of the present disclosure relates to use of the compound as described above in an analytical assay to identify the capacity of other candidate compounds to inhibit one or more kinases, more preferably LRRK, and even more preferably LRRK2.

Preferably, the analytical assay is a competitive binding assay.

More preferably, the competitive binding assay comprises contacting a compound of the present disclosure with a kinase, preferably LRRK, and more preferably LRRK2 and a candidate compound, and detecting any change in the interaction between the compound according to the present disclosure and the kinase.

Preferably, the candidate compound is generated by SAR modification of the compound of the present disclosure.

As used herein, the term "SAR modification" refers to a standard method of altering a given compound by chemical derivatization.

Thus, in one aspect, the identified compound can be used as a model (e.g., template) for the development of other compounds. The compounds used in such assays may be free in solution, fixed onto a solid carrier, supported on the surface of cells, or located in the cells. The elimination of activity or the formation of a binding complex between a compound and a medicament to be tested can be measured.

The analytical assay of the present disclosure may be a screening, whereby a large number of medicaments are tested. In one aspect, the analytical assay method of the present disclosure is a high-throughput screening.

The present disclosure also contemplates the use of competitive drug screening assays in which neutralizing antibodies capable of binding to a compound specifically compete with a test compound for binding to the compound.

Another technique for screening is provided for high-throughput screening (HTS) of agents with suitable binding affinity for a substance and is based on the method described in detail in WO 84/03564.

It is contemplated that the analytical assay method of the present disclosure will be suitable for small-scale and large-scale screening of test compounds as well as for quantitative assays.

Preferably, the competitive binding assay comprises contacting a compound of the present disclosure with a kinase in the presence of a known substrate for the kinase, and detecting any change in the interaction between the kinase and the known substrate.

Another aspect of the present disclosure provides a method for detecting the binding of a ligand to a kinase, the method comprising the following steps:
(i) contacting a ligand with a kinase in the presence of a known substrate for the kinase; and
(ii) detecting any change in an interaction between the kinase and the known substrate; wherein the ligand is a compound of the present disclosure.

One aspect of the present disclosure relates to a method comprising the following steps:
(a) performing the assay method described above;
(b) identifying one or more ligands capable of binding to a ligand binding domain; and
(c) preparing an amount of the one or more ligands.

Another aspect of the present disclosure provides a method comprising the following steps:
(a) performing the assay method described above;
(b) identifying one or more ligands capable of binding to a ligand binding domain; and
(c) preparing a pharmaceutical composition comprising the one or more ligands.

Another aspect of the present disclosure provides a method comprising the following steps:
(a) performing the assay method described above;
(b) identifying one or more ligands capable of binding to a ligand binding domain;
(c) modifying the one or more ligands capable of binding to the ligand binding domain;
(d) performing the assay method described above; and
(e) optionally preparing a pharmaceutical composition comprising the one or more ligands.

The present disclosure further relates to a ligand identified by the method described above.

Another aspect of the present disclosure relates to a pharmaceutical composition comprising a ligand identified by the method described above.

Another aspect of the present disclosure relates to use of a ligand identified by the method described above for the manufacturing of a pharmaceutical composition for the treatment of one or more disorders.

The method described above may be used for screening for ligands that may be used as inhibitors of one or more kinases.

### BRIEF DESCRIPTION OF THE DRAWINGS

FIG. 1 shows the mean plasma concentration-time curves for mice after administration in Example 5; and
FIG. 2 shows the drug concentration-time curves for the brain tissue of mice after administration in Example 5.

### DETAILED DESCRIPTION

The technical scheme of the present disclosure will be further illustrated in detail with reference to the following specific examples. It should be understood that the following examples are merely exemplary illustration and explanation of the present disclosure, and should not be construed as limiting the protection scope of the present disclosure. All techniques implemented based on the content of the present disclosure described above are encompassed within the protection scope of the present disclosure.

Unless otherwise stated, the starting materials and reagents used in the following examples are all commercially available products or can be prepared using known methods.

The following abbreviations are used throughout the specification:
- AcOH: acetic acid
- DCM: dichloromethane
- DEA: diethylamine
- DMF: *N,N*-dimethylformamide
- EA, EtOAc: ethyl acetate
- IPA: isopropanol
- NMR: nuclear magnetic resonance
- PE: petroleum ether
- SFC: supercritical fluid chromatography
- TEA: triethylamine
- TFA: trifluoroacetic acid
- THF: tetrahydrofuran
- TMS: trimethylsilyl
- TMSOTf: trimethylsilyl trifluoromethanesulfonate
- t*_{R}*: retention time
- Ts: p-toluenesulfonyl
- UV: ultraviolet ray

### Chromatography

High pressure liquid chromatography is performed using equipment manufactured by Agela Technologies and monitored by a multi-wavelength UV detector. Typical mobile phases for the separation process are PE/EA, DCM/MeOH or water/MeCN.

### Analytical method

¹H nuclear magnetic resonance (NMR) spectroscopy is performed at room temperature in the solvent using Bruker AV 400 spectrometer unless otherwise stated. In all cases, the NMR data are consistent with the proposed structure. Using conventional abbreviations for designating the main peaks, the characteristic chemical shifts (δ) are given in parts per million: for example, s, singlet; d, doublet; t, triplet; q, quartet; dd, doublet of doublets; br, wide. Mass spectra are recorded using Agilent 1290 Infinity/6460 triple Quad LCMS. When thin-layer chromatography (TLC) is used, it refers to silica gel TLC.

### Preparation of Compounds

Without the description of the preparation of starting materials, these starting materials may be purchased commercially, known in the literature, or readily available to those skilled in the art using standard procedures. In the case where compounds are illustrated as being prepared through analogy to previous examples or intermediates, it will be appreciated by those skilled in the art that the reaction time, number of equivalents of reagents, and temperature for each particular reaction can be varied, and that it may be necessary or desirable to employ different post-treatment or purification techniques.

### Example 1

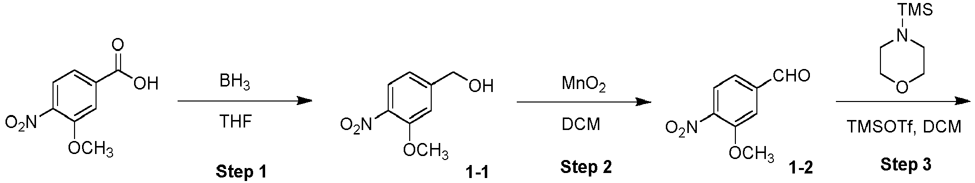

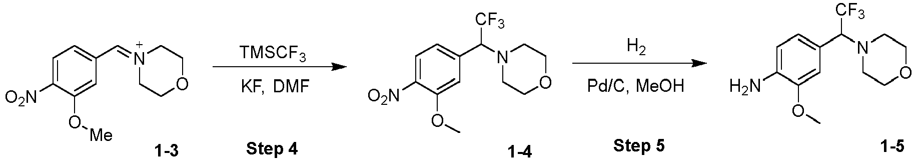

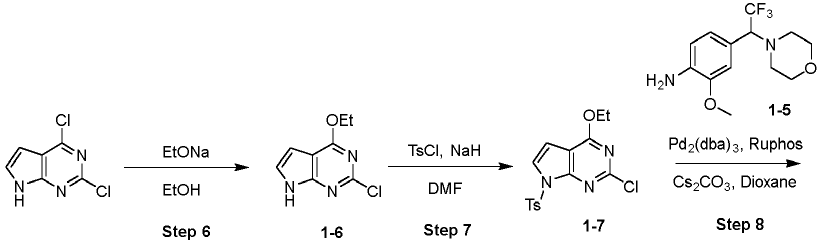

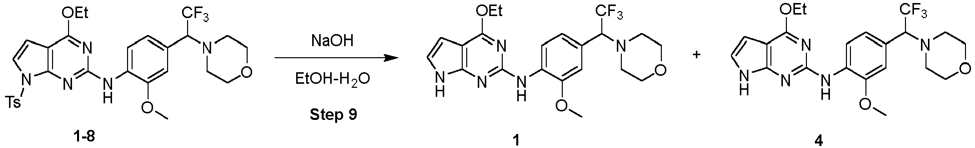

### Step 1: (3-methoxy-4-nitrophenyl)methanol (intermediate 1-1)

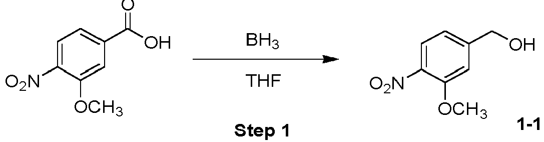

To a solution of 3-methoxy-4-nitrobenzoic acid (15.0 g, 0.0761 mol) in THF (50 mL) was added dropwise a solution of BH₃ in THF (228 mL, 0.228 mol, 1 M) under an ice bath at 0 °C. The resulting mixture was heated to 60 °C and stirred for 4 h. After the mixture was cooled to 0 °C, MeOH (30 mL) was added to quench the reaction. The resulting mixture was concentrated to give a residue, which was then purified by silica gel column chromatography (PE/EA = 1:1) to give (3-methoxy-4-nitrophenyl)methanol (1-1, 10.0 g, 71.9%) as a yellow solid, m/z (ESI)⁺: 184 [M+H]⁺.

### Step 2: 3-methoxy-4-nitrobenzaldehyde (intermediate 1-2)

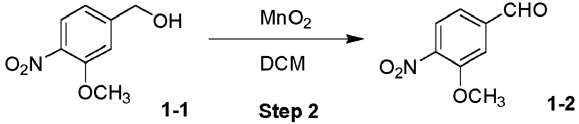

A mixture of intermediate 1-1 (10.0 g, 54.6 mmol) and MnO₂ (9.48 g, 109 mmol) was refluxed overnight in DCM (200 mL). The mixture was cooled to room temperature and then filtered through celite, and the filtrate was concentrated to give 3-methoxy-4-nitrobenzaldehyde (1-2, 6.67 g, 67.4%) as a white solid. ¹H NMR (300 MHz, CDCl₃) *δ* 10.05 (s, 1H), 7.92 (d, *J* = 8.1 Hz, 1H), 7.60 (d, *J* = 1.3 Hz, 1H), 7.54 (dd, *J=* 8.1, 1.4 Hz, 1H), 4.03 (s, 3H). m/z (ESI)⁺: 182 [M+H]⁺.

### Step 3: 4-(3-methoxy-4-nitrobenzylidene)morpholin-4-yl (intermediate 1-3)

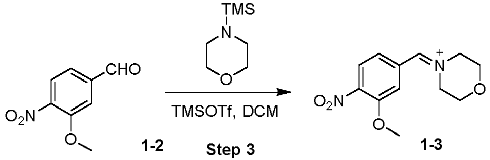

To a solution of intermediate 1-2 (1.00 g, 5.52 mmol) and 4-(trimethylsilyl)morpholine (1.05 g, 6.60 mmol) in DCM (25 mL) was added dropwise TMSOTf (1.5 g, 6.60 mmol) at 0 °C. The resulting mixture was stirred at room temperature for 2 h and then concentrated to give crude intermediate 1-3, which was used directly in the next step.

### Step 4: 4-(2,2,2-trifluoro-l-(3-methoxy-4-nitrophenyl)ethyl)morpholine (intermediate 1-4)

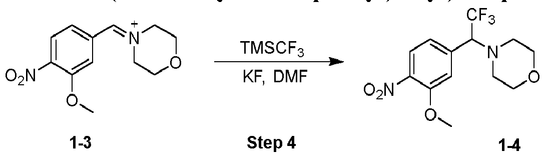

To a solution of crude intermediate 1-3 (about 5.5 mmol) in DMF (20 mL) were added sequentially TMSCF₃ (1.18 g, 8.25 mmol) and KF (0.50 g, 8.25 mmol) at 0 °C. When the reaction solution became red, the reaction system was heated to room temperature and stirred overnight. The reaction mixture was diluted with EtOAc (100 mL), washed with brine (30 mL), dried over anhydrous Na₂SO₄, filtered, and concentrated to give a residue. The residue was purified by silica gel column chromatography (PE/EA = 5:1) to give 4-(2,2,2-trifluoro-1-(3-methoxy-4-nitrophenyl)ethyl)morpholine (1-4, 1.00 g, 56% over two steps) as a white solid, m/z (ESI)⁺: 321 [M+H]⁺.

### Step 5: 2-methoxy-4-(2,2,2-trifluoro-1-morpholinoethyl)aniline (intermediate 1-5)

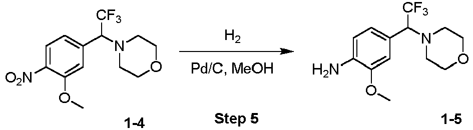

A mixture of intermediate 1-4 (1.00 g, 3.12 mmol) and 10% Pd/C (300 mg) in MeOH (30 mL) was degassed and purged 3 times with H₂. The resulting mixture was then stirred at room temperature under H₂ atmosphere overnight. The resulting mixture was filtered through celite, and the filtrate was concentrated to give crude 2-methoxy-4-(2,2,2-trifluoro-1-morpholinoethyl)aniline (1-5, 800 mg) as a white solid, m/z (ESI)⁺: 291 [M+H]⁺.

### Step 6: 2-chloro-4-ethoxy-7H-pyrrolo[2,3-d|pyrimidine (intermediate 1-6)

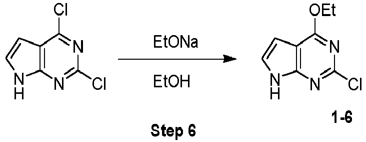

To a solution of 2,4-dichloro-7*H*-pyrrolo[2,3-*d*]pyrimidine (10.0 g, 0.054 mol) in EtOH (300 mL) was added EtONa (3.6 g, 0.054 mol). The resulting mixture was stirred at 90 °C overnight. The reaction mixture was concentrated, diluted with EtOAc (300 mL), and washed with brine. The separated organic layer was dried over anhydrous Na₂SO₄, filtered, and concentrated to give a crude product. The residue was purified by flash column chromatography (PE/EA = 20:1) to give crude 2-chloro-4-ethoxy-7*H*-pyrrolo[2,3-*d*]pyrimidine (**1-6,** 5.0 g) as a white solid, m/z (ESI)⁺: 198 [M+H]⁺.

### Step 7: 2-chloro-4-ethoxy-7-tosyl-7H-pyrrolo[2,3-d]pyrimidine (intermediate 1-7)

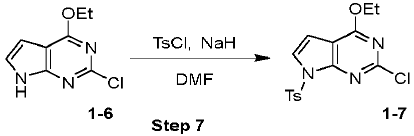

To a solution of crude intermediate 1-6 (5.0 g, 25 mmol) in DMF (20 mL) was added NaH (1.22 g, 30.4 mmol) at 0 °C. The resulting mixture was stirred at 0 °C for 0.5 h. TsCl (5.80 g, 30.4 mmol) was then added at 0 °C. The resulting mixture was stirred at room temperature for 2 h. Water was added to quench the reaction at 0 °C, and EtOAc (200 mL) was then added for dilution. The organic layer was washed with brine, dried over anhydrous Na₂SO₄, filtered, and concentrated to give a crude product. The resulting residue was purified by flash column chromatography (PE/EA = 20:1) to give 2-chloro-4-ethoxy-7-tosyl-7H-pyrrolo[2,3-d]pyrimidine (1-7, 2.00 g, 22% over two steps). m/z (ESI)⁺: 352 [M+H]⁺.

### Step 8: 4-ethoxy-N-(2-methoxy-4-(2,2,2-trifluoro-l-morpholinoethyl)phenyl)-7-tosyl-7H-pyrrolo[2,3-d]pyrimidin-2-amine (intermediate 1-8)

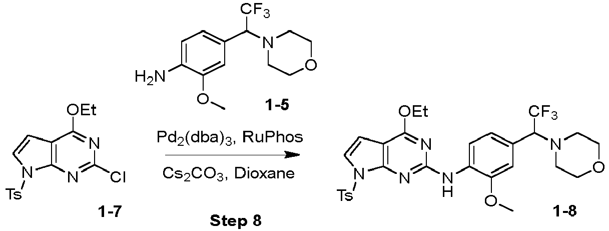

To a solution of intermediate 1-7 (1.20 g, 3.42 mmol) and intermediate 1-5 (1.00 g, 3.44 mmol) in 1,4-dioxane (10 mL) were added Pd₂(dba)₃ (275 mg, 0.310 mmol), RuPhos (280 mg, 0.617 mmol), and Cₛ₂CO₃ (1.95 g, 5.98 mmol) under N₂ atmosphere. The resulting mixture was stirred in a microwave reactor at 125 °C for 1 h. The reaction mixture was cooled to room temperature and then diluted with EtOAc and water. The separated organic layer was washed with brine, dried over anhydrous Na₂SO₄, filtered, and concentrated to give a residue. The residue was purified by silica gel column chromatography (EA) to give 4-ethoxy-N-(2-methoxy-4-(2,2,2-trifluoro-1-morpholinoethyl)phenyl)-7-tosyl-7H-pyrrolo[2,3-d]pyrimidin-2-amine (1-8, 900 mg, 43.6%). m/z (ESI)⁺: 606 [M+H]⁺.

### Step 9: Synthesis of 4-ethoxy-N-(2-methoxy-4-(2,2,2-trifluoro-1-morpholinoethyl)phenyl)-17H-pyrrolo[2,3-d]pyrimidin-2-amine (enantiomer 1, compound 1) and 4-ethoxy-N-(2-methoxy-4-(2,2,2-trifluoro-1-morpholinoethyl)phenyl)-7H-pyrrolo[2,3-d]pyrimidin-2-amine (enantiomer 2, compound 4)

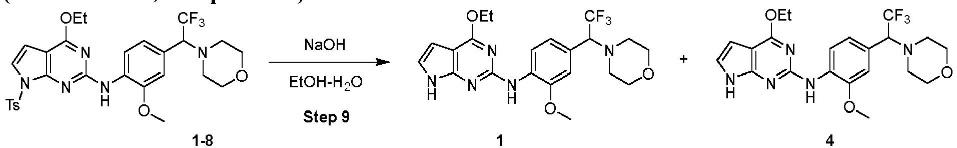

To a solution of intermediate 1-8 (900 mg, 1.49 mmol) in EtOH/H₂O (10 mL/1 mL) was added NaOH (297 mg, 7.43 mmol). The resulting mixture was stirred at 50 °C for 2 h. The mixture was concentrated to give a residue. The residue was purified by flash column chromatography (EA:PE = 1:1) to give an enantiomeric mixture of 4-ethoxy-N-(2-methoxy-4-(2,2,2-trifluoro-1-morpholinoethyl)phenyl)-7H-pyrrolo[2,3-d]pyrimidin-2-amine (800 mg), and separated by supercritical fluid chromatography (SFC) (chiralpak-IC column, 4.6 mm × 250 mm, 5 µm, eluted with CO₂-IPA (0.2% DEA)) to give **compound** 1 (enantiomer 1, 310 mg, 38.8%) and **compound 4** (enantiomer 2, 300 mg, 37.5%) as white solids.

**Compound** 1: ¹H NMR (400 MHz, CD₃OD) *δ* 8.66 (d, *J* = 8.3 Hz, 1H), 7.24-6.89 (m, 3H), 6.38 (d, *J=* 3.5 Hz, 1H), 4.59 (q, *J=* 7.1 Hz, 2H), 4.22-4.07 (m, 1H), 3.98 (s, 3H), 3.70 (t, *J=* 4.7 Hz, 4H), 2.67-2.58 (m,4H), 1.50 (t, *J=* 7.1 Hz, 3H); ¹⁹F-NMR (400 MHz, CD₃OD) *δ* 68.40 ppm; m/z (ESI)⁺: 452 [M+H]⁺, t*_{R}* = 1.94 min, optical rotation: -52.5° (1 mg/mL of ethanol solution);

**Compound 4:** ¹H NMR (400 MHz, CD₃OD) *δ* 8.66 (d, *J=* 8.3 Hz, 1H), 7.24-6.89 (m, 3H), 6.38 (d, *J=* 3.5 Hz, 1H), 4.59 (q, *J=* 7.1 Hz, 2H), 4.22-4.07 (m, 1H), 3.98 (s, 3H), 3.70 (t, *J=* 4.7 Hz, 4H), 2.67-2.58 (m,4H), 1.50 (t, *J=* 7.1 Hz, 3H); ¹⁹F-NMR (400 MHz, CD₃OD) *δ* 68.40 ppm; m/z (ESI)⁺: 452 [M+H]⁺, t*_{R}* = 2.33 min, optical rotation: +43.5° (1 mg/mL of ethanol solution).

Referring to Example 1, by using suitable starting materials and reagents of steps 1, 3 or 6, compounds 5 to 34 as shown in Table 1 were prepared.

**Table 1**

| **Compound** | **Structure** | **m/z (ESI)⁺ [M+H]⁺** | t*_{R}* **(min)***^{a}* |
|---|---|---|---|
| 5 (Enantiomer 1) | 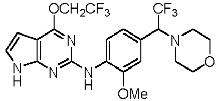 | 506 | 2.46 |
| 6 (Enantiomer 2) | 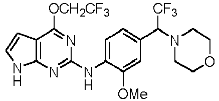 | 506 | 2.77 |
| 7 (Enantiomer 1) | 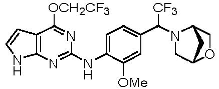 | 518 | 1.54 |
| 8 (Enantiomer 2) | 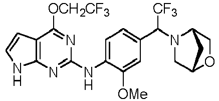 | 518 | 1.78 |
| 9 (Enantiomer 1) | 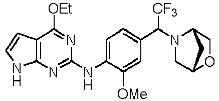 | 464 | 3.08 |
| **10** (Enantiomer 2) | 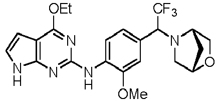 | 464 | 3.73 |
| 11 (Enantiomer 1) | 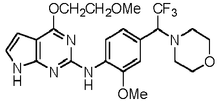 | 482 | 2.42 |
| **12** (Enantiomer 2) | 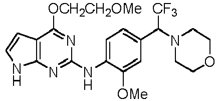 | 482 | 2.84 |
| 13 (Enantiomer 1) | 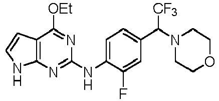 | 440 | 8.78*^{b}* |
| **14** (Enantiomer 2) | 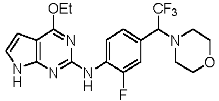 | 440 | 9.87*^{b}* |
| **15** (Enantiomer 1) | 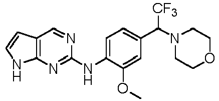 | 408 | 5.66 |
| **16** (Enantiomer 2) | 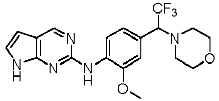 | 408 | 6.21 |
| **17** (Enantiomer 1) | 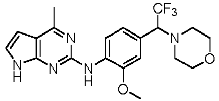 | 422 | 2.54 |
| **18** (Enantiomer 2) | 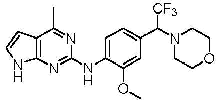 | 422 | 2.85 |
| **19** (Enantiomer 1) | 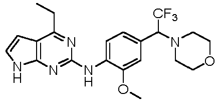 | 436 | 1.70 |
| **20** (Enantiomer 2) | 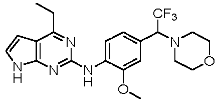 | 436 | 1.82 |
| **21** (Enantiomer 1) | 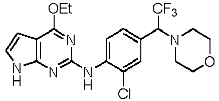 | 456 | 3.32 |
| **22** (Enantiomer 2) | 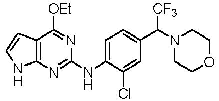 | 456 | 3.60 |
| **23** (Enantiomer 1) | 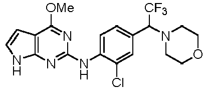 | 442 | 3.31 |
| **24** (Enantiomer 2) | 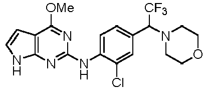 | 442 | 3.52 |
| **25** (Enantiomer 1) | 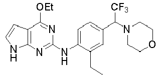 | 450 | 3.12 |
| **26** (Enantiomer 2) | 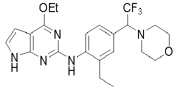 | 450 | 3.63 |
| **27** (Enantiomer 1) | 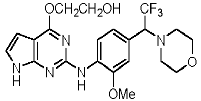 | 468 | 2.06 |
| **28** (Enantiomer 2) | 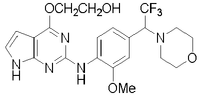 | 468 | 2.44 |
| **29** (Enantiomer 1) | 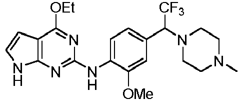 | 465 | 3.40 |
| **30** (Enantiomer 2) | 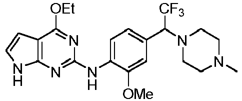 | 465 | 3.83 |
| **31** (Enantiomer 1) | 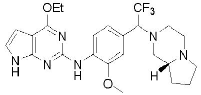 | 491.1 | 1.79 |
| **32** (Enantiomer 2) | 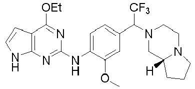 | 491 | 1.91 |
| **33** (Enantiomer 1) | 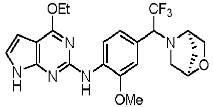 | 463.9 | 2.80 |
| **34** (Enantiomer 2) | 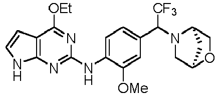 | 463.8 | 3.15 |

| | | | |
|---|---|---|---|
| *^{a}*: SFC, chiralpak-IC column, 4.6 mm × 250 mm, 5 µm, eluted with CO₂-IPA (0.2% DEA). *^{b}*: OD-H column, 20 mm × 250 mm, 5 µm, eluted with *n*-hexane/IPA (0.2% NH₄OH) = 80:20. | | | |

### Example 2

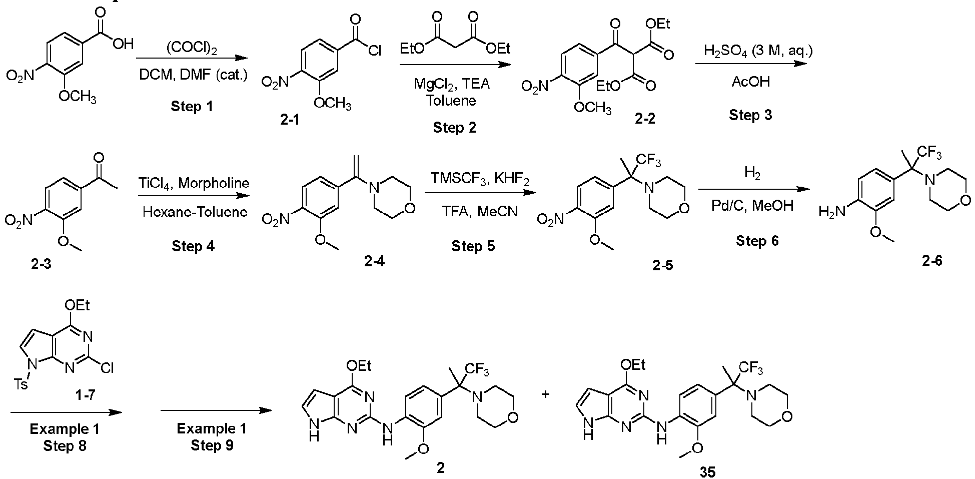

### Step 1: 3-methoxy-4-nitrobenzoyl chloride (intermediate 2-1)

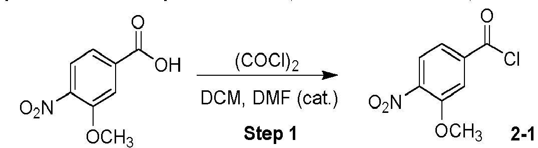

To a solution of 3-methoxy-4-nitrobenzoic acid (5.00 g, 25.4 mmol) in DCM (100 mL) was added dropwise a few drops of DMF. A solution of oxalyl chloride (6.50 g, 51.0 mmol) in DCM (20 mL) was then added dropwise under an ice bath at 0 °C. The resulting mixture was heated to room temperature and stirred for 2 h. The mixture was concentrated to give 3-methoxy-4-nitrobenzoyl chloride (2-1, 5.40 g, 98.8%) as a yellow solid, which was used in the next step without further purification.

### Step 2: diethyl 2-(3-methoxy-4-nitrobenzoyl)malonate intermediate 2-2)

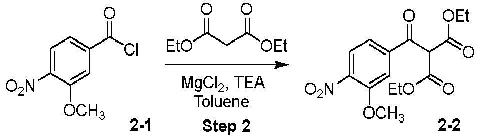

A suspension of MgCl₂ (1.67 g, 17.6 mmol) suspended in toluene (50 mL) was treated with TEA (6.10 g, 60.2 mmol) and diethyl malonate (4.80 g, 30.1 mmol) at room temperature. The mixture was stirred at room temperature for 1.5 h. Intermediate 2-1 (5.40 g, 25.1 mmol) was then added in batches and stirred for 1 h. The reaction mixture was added with 1.0 M HCl (10 mL) under stirring and then extracted with EtOAc (100 mL × 2). The combined organic layers were washed with brine, dried over anhydrous Na₂SO₄, filtered, and concentrated to give crude 2-(3-methoxy-4-nitrobenzoyl)malonate (2-2, 10.5 g) as a yellow solid, m/z (ESI)⁺: 340 [M+H]⁺.

### Step 3: 1-(3-methoxy-4-nitrophenyl)ethane-1-one (intermediate 2-3)

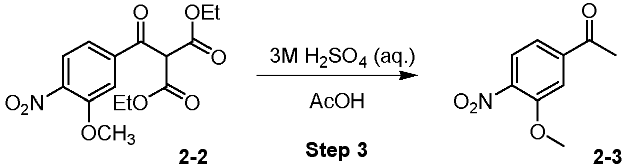

A solution of crude intermediate 2-2 (*ca* 25.1 mmol) in acetic acid (50 mL) and 3 M H₂SO₄ (aq., 50 mL) were heated to 120 °C and stirred overnight. The resulting solution was cooled to room temperature and diluted with EtOAc (200 mL). The separated organic phase was washed with brine, dried over anhydrous Na₂SO₄, filtered, and concentrated to give a residue. The residue was purified by silica gel column chromatography (PE/EA = 3:2) to give 1-(3-methoxy-4-nitrophenyl)ethan-1-one (2-3, 3.60 g, 72% over two steps) as an off-white solid, m/z (ESI)⁺: 196 [M+H]⁺.

### Step 4: 4-(1-(3-methoxy-4-nitrophenyl)vinyl)morpholine (intermediate 2-4)

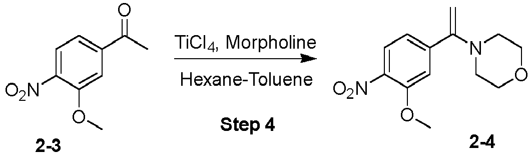

To a solution of morpholine (1.34 g, 15.4 mmol) in hexane (20 mL) was added a solution of TiCl₄ in DCM (1 M, 2.3mL, 2.3 mmol) at 0 °C under N₂ atmosphere. A solution of intermediate 2-3 (500 mg, 2.56 mmol) in toluene (10 mL) was then added dropwise, and the resulting mixture was heated to 70 °C and stirred for 20 min. The mixture was cooled to room temperature and filtered through celite. The filtrate was concentrated to give crude 4-(1-(3-methoxy-4-nitrophenyl)vinyl)morpholine (2-4, 670 mg) as a yellow oil. ¹H NMR (300 MHz, CDCl₃) δ 7.85 (d, *J =* 8.1 Hz, 1H), 7.19 (s, 1H), 6.14 (d, *J* = 8.4 Hz, 1H), 4.46 (d, *J* = 6.6 Hz, 2H), 3.98 (s, 3H), 3.79-3.76 (m, 4H), 2.84-2.80 (m, 4H).

### Step 5: 4-(1,1,1-trifluoro-2-(3-methoxy-4-nitrophenyl)propan-2-yl)morpholine (intermediate 2-5)

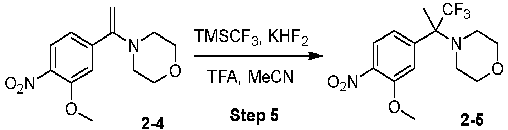

To a solution of crude intermediate 2-4 (570 mg, 2.15 mmol) and KHF₂ (168 mg, 2.15 mmol) in MeCN (30 mL) was added TFA (368 mg, 4.30 mmol) at 0 °C under N₂ atmosphere, followed by the addition of TMSCF₃ (610 mg, 8.25 mmol). The reaction mixture was then heated to room temperature and stirred overnight. The reaction mixture was quenched with Na₂CO₃ (aq., 20 mL) and extracted with EA (50 mL). The separated organic phase was washed with brine (30 mL), dried over anhydrous Na₂SO₄, filtered, and concentrated to give a residue. The residue was purified by silica gel column chromatography (PE/EA = 7:3) to give 4-(1,1,1-trifluoro-2-(3-methoxy-4-nitrophenyl)propan-2-yl)morpholine (2-5, 200 mg, 27%) as a yellow solid, m/z (ESI)⁺: 335 [M+H]⁺.

### Step 6: 2-methoxy-4-(1,1,1-trifluoro-2-morpholinopropan-2-yl)aniline (intermediate 2-6)

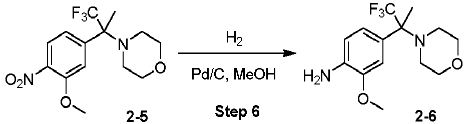

A mixture of intermediate 5 (200 mg, 0.60 mmol) and 10% Pd/C (100 mg) in MeOH (30 mL) was degassed and purged 3 times with H₂. The resulting mixture was then stirred at room temperature under H₂ atmosphere overnight. The resulting mixture was filtered through celite and then concentrated to give crude 2-methoxy-4-(1,1,1-trifluoro-2-morpholinopropan-2-yl)aniline (6, 150 mg) as a white solid, m/z (ESI)⁺: 305 [M+H]⁺.

### Step 7: 4-ethoxy-N-(2-methoxy-4-(1,1,1-trifluoro-2-morpholinylpropan-2-yl)phenyl)-7H-pyrrolo[2,3-d]pyrimidin-2-amine (enantiomer 1, compound 2) and 4-ethoxy-N-(2-methoxy-4-(1,1,1-trifluoro-2-morpholinopropan-2-yl)phenyl)-7H-pyrrolo[2,3-d]pyrimidin-2-amine enantiomer 2, compound 35)

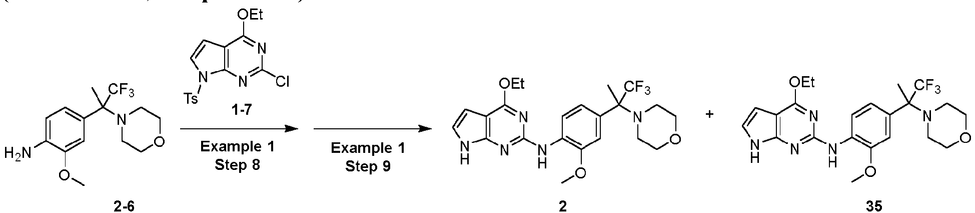

Similar to steps 8-9 in Example 1, compound 2 was synthesized from intermediate 2-6: 4-ethoxy-N-(2-methoxy-4-(1,1,1-trifluoro-2-morpholinylpropan-2-yl)phenyl)-7*H*-pyrrolo[2,3-*d*]pyrimidin-2-amine (enantiomer 1, t*_{R}* = 1.63 min) and compound 35: 4-ethoxy-*N*-(2-methoxy-4-(1,1,1-trifluoro-2-morpholinylpropan-2-yl)phenyl)-7H-pyrrolo[2,3-d]pyrimidin-2-amine (enantiomer 2, t*_{R}* = 2.11 min). **Compound** 2: ¹HNMR (400 MHz, CD₃OD) *δ* 8.58 (d, J= 8.6 Hz, 1H), 7.32 (s, 1H), 7.15 (d, J= 8.6 Hz, 1H), 6.92 (d, *J* = 3.5 Hz, 1H), 6.36 (d, *J* = 3.5 Hz, 1H), 4.56 (q, *J* = 7.1 Hz, 2H), 3.95 (s, 3H), 3.68 (t, *J =* 4.4 Hz, 4H), 2.75-2.58 (m, 4H), 1.60 (s, 3H), 1.47 (t, *J =* 7.1 Hz, 3H); ¹⁹F-NMR (400 MHz, CD₃OD) *δ* 68.97 ppm; m/z (ESI)⁺: 466 [M+H]⁺. **Compound 35:** ¹H NMR (400 MHz, CD₃OD) *δ* 8.62-8.52 (m, 1H), 7.32 (s, 1H), 7.15 (d, *J=* 8.7 Hz, 1H), 6.92 (d, *J* = 3.5 Hz, 1H), 6.36 (d, *J=* 3.5 Hz, 1H), 4.56 (q, *J* = 7.1 Hz, 2H), 3.95 (s, 3H), 3.68 (t, *J* = 4.4 Hz, 4H), 2.75-2.58 (m,4H), 1.60 (s, 3H), 1.47 (t, *J* = 7.1 Hz, 3H); ¹⁹F-NMR (400 MHz, CD₃OD) *δ* 68.97 ppm; m/z (ESI)⁺: 466 [M+H]⁺.

### Example 3

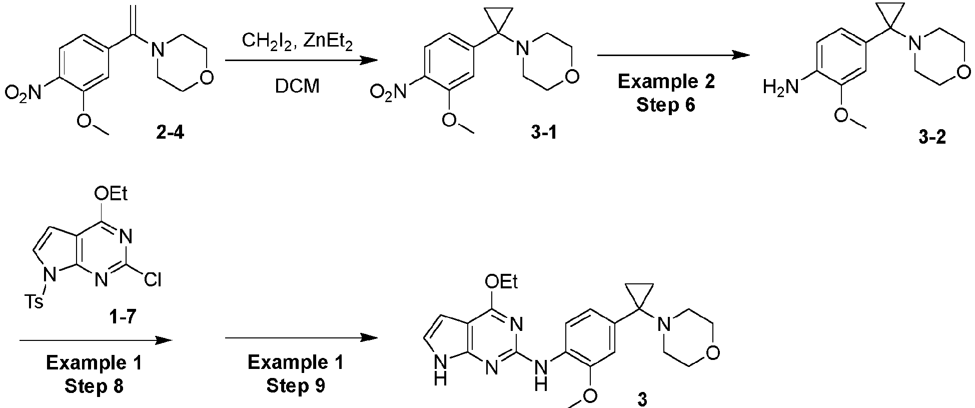

### Step 1: 4-(1-(3-methoxy-4-nitrophenyl)cyclopropyl)morpholine (intermediate 3-1)

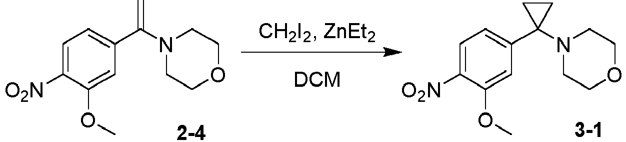

To a mixture of CH₂I₂ (1.01 g, 3.77 mmol) and a solution of crude intermediate 2-4 (500 mg, 1.89 mmol) in DCM (50 mL) was added dropwise a solution of ZnEt₂ in hexane (1 M, 2.8 mL, 2.80 mmol) at 0 °C under N₂ atmosphere. The mixture was stirred at this temperature for 3 h. NH₄Cl (aq., 10 mL) was added to the reaction mixture. The separated organic layer was dried over anhydrous Na₂SO₄, filtered, and concentrated to give a residue. The residue was purified by silica gel column chromatography (PE/EA = 1: 1) to give 4-(1-(3-methoxy-4-nitrophenyl)cyclopropyl)morpholine (3-1, 500 mg, 95%) as a yellow solid, m/z (ESI)⁺: 279 [M+H]⁺.

### Step 2: 2-methoxy-4-(1-morpholinocyclopropyl)aniline (intermediate 3-2)

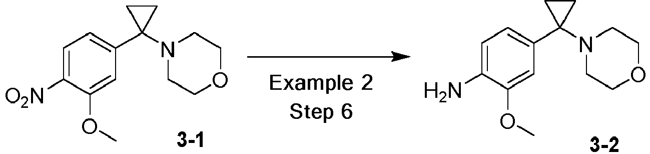

Similar to step 6 in Example 2, 2-methoxy-4-(1-morpholinocyclopropyl)aniline (intermediate 3-2) was prepared from intermediate 3-1. m/z (ESI)⁺: 249 [M+H]⁺.

### Step 3: 4-ethoxy-N-(2-methoxy-4-(1-morpholinocyclopropyl)phenyl)-7H-pyrrolo[2,3-d]pyrimidin-2-amine (compound 3)

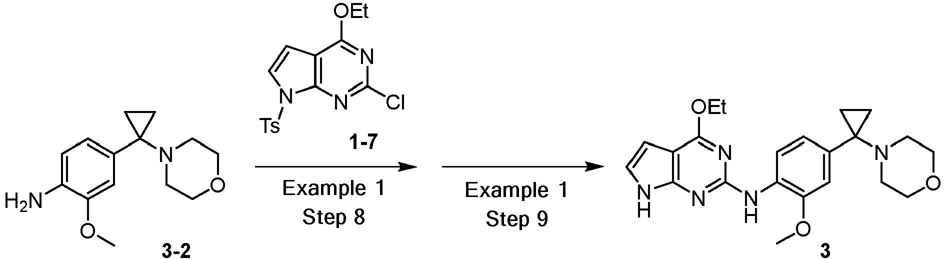

Similar to steps 8-9 in Example 1, 4-ethoxy-*N*-(2-methoxy-4-(1-morpholinocyclopropyl)phenyl)-7H-pyrrolo[2,3-d]pyrimidin-2-amine (compound 3) was synthesized from intermediate 3-2. ¹H NMR (400 MHz, CD₃OD) *δ* 8.53 (d, *J=* 8.7 Hz, 1H), 6.91-6.89 (m, 3H), 6.35 (d, *J* = 3.5 Hz, 1H), 4.56 (q, *J* = 7.1 Hz, 2H), 3.95 (s, 3H), 3.61 (t, *J* = 4.8 Hz, 4H), 2.67-2.58 (m,4H), 1.47 (t, *J=* 7.1 Hz, 3H), 0.96-0.93 (m, 2H), 0.84-0.81 (m, 2H); m/z (ESI)⁺: 410 [M+H]⁺.

Referring to Examples 1 and 3, compounds 36 to 38 as shown in Table 2 were synthesized by using suitable steps, starting materials and reagents.

**Table 2**

| **Compound** | **Structure** | **m/z(ESI)⁺ [M+H]⁺** |
|---|---|---|
| **36** | 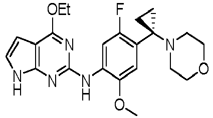 | 427.8 |
| **37** | 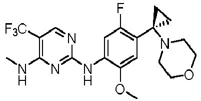 | 441.8 |
| **38** | 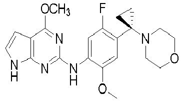 | 413.9 |

### Example 4: Kinase Inhibition Assay

### Material

In the Adapta^{™} kinase assay method (LRRK2_IC₅₀ assay): the kinase reaction buffer contained 5× kinase buffer S (Life Technologies, PV5213) and 2 mM DTT (Life Technologies, P2325). The kinases LRRK2 G2019S protein (PV4881) and ERM (LRRKtide, PV5093) were derived from Life Technologies. The Adapta^{™} universal kinase assay kit (Life Technologies, PV5099) contained the following components: Adapta^{™} Eu-anti-ADP antibody (PV5097; 4 µg); 10 µM AlexaFluor^{®}647 ADP tracer (PV5098; 200 pmol); TR-FRET dilution buffer (PV3574; 100 mL); kinase quench buffer (P2825; 1 mL); 10 mM ATP (PV3227; 500 µL); and 10 mM ADP (PV5096; 500 µL). LRRK2-IN-1 (1234480-84-2, HY-10875) was derived from MedChem Express.

### Adapta^{™} kinase assay method

Adapta^{®} universal kinase assay method is a uniform fluorescence-based immunoassay for detecting ADP. In contrast to ATP consumption assay, Adapta^{®} assay method was very sensitive to ADP formation, with most signal changes occurring when initially 10-20% ATP was converted to ADP. This made Adapta^{®} universal kinase assay method very suitable for low-activity kinases.

All assays were performed at room temperature (about 21 °C) and were linearly related to time and enzyme concentration under the conditions used. 1× kinase reaction buffer was prepared from 5× kinase buffer S stock solution (listed above), and 10 mL of 1× kinase reaction buffer was prepared by the addition of 2 mL of 5× stock solution to 8 mL of H₂O. To the 1× kinase reaction buffer was added 20 µL of 1 M DTT.

A kinase reaction was performed in a low-volume 384-well plate in a volume of 10 µL. Typically, a Greiner plate was used (Catalog #3674#, low volume, white wall, 784075). Other untreated assay plates that were not tested could also be suitable. The substrate concentration in the assay was 100 µM, and the 1× kinase reaction buffer consisted of 50 mM Tris-HCl (pH 8.5), 10 mM MgCl₂, 1 mM EGTA, 0.01% Brij-35 and 2 mM DTT, as well as any other additives that could be necessary for the particular kinase. The kinase reaction was allowed to proceed for 1 h at room temperature, followed by the addition of a formulation of kinase quenching buffer (EDTA; 30 mM), Eu-labeled antibody (6 nM), and tracer (18.9 nM) in TR-FRET dilution buffer (5 µL). The final concentration of the antibody in the assay wells was 2 nM, the final concentration of the tracer was 6.3 nM, and the final concentration of EDTA was 10 mM. The plate was allowed to equilibrate at room temperature for at least 30 min and then read on a plate reader configured for Adapta^{™} TR-FRET.

The data provided in this document were generated by using BMG LABTECH PHERAstar plate reader and using appropriate filters and instrument settings for Adapta^{™}. Test compounds were screened in wells with 1% DMSO (final). For 8-point titration, a 5-fold serial dilution was made on the basis of a starting concentration.

### Results

The IC₅₀ values for LRRK2 G2019S inhibition by compounds of the examples of the present disclosure are shown in Table 3 below.

**Table 3**

| **Compound** | **LRRK2 IC₅₀** (G2019S, nM) |
|---|---|
| **1** | 12 |
| **4** | 17 |
| **5** | 17 |
| **6** | 12 |
| **7** | 47 |
| **8** | 52 |
| **9** | 7 |
| **10** | 11 |
| **11** | 6 |
| **12** | 12 |
| **13** | 31 |
| **14** | 30 |
| **15** | 328 |
| **16** | 430 |
| **17** | 204 |
| **18** | 253 |
| **19** | 332 |
| **20** | 235 |
| **21** | 119 |
| **22** | 138 |
| **23** | 147 |
| **24** | 122 |
| **25** | 141 |
| **26** | 70 |
| **27** | 5 |
| **28** | 5 |
| **29** | 12 |
| **30** | 22 |
| **31** | 64 |
| **32** | 27 |
| **33** | 10 |
| **34** | 8 |
| **2** | 55 |
| **35** | 275 |
| **3** | 7 |
| **36** | 38 |
| **37** | 34 |
| **38** | 25 |

### Example 5: Drug Tissue Distribution Assay

### Test sample: compound 4, purity 99%

Preparation of test sample: 22.73 mg of compound 4 was weighed and placed in a mortar (according to the purity of 99%), and a small amount of 0.5% CMC-Na was added followed by full grinding. The mixture was then transferred to a container; the mortar was washed for 4 times; the washing solution was transferred to the container, followed by the addition of 0.5% CMC-Na until the volume of the reaction solution was 30 mL; and then a test sample suspension with a concentration of 0.75 mg/mL was prepared. The suspension was stirred with a magnetic stirrer at a rotation speed of 1200 rpm for at least 15 min prior to sampling for analysis and administration, with fresh preparation just prior to use.

### Route of administration: oral intragastric administration

### Frequency and dose of administration: single administration at 30 mg/kg (40 mL/kg)

After drug administration, 0.4 mL of blood was collected from the heart of each group of animals at corresponding time points of 0.25 h, 0.5 h, 1 h, 2 h, 4 h, 6 h, 8 h, and 24 h, respectivley, the animals were anesthetized by intraperitoneal injection of 50 mg/kg Zoletil, and then put to death by bleeding. The brain, lung, and kidney of each animal were taken out, washed with normal saline, dried off with filter paper, and put into a cryopreservation tube. The cryopreservation tube was temporarily placed in dry ice, and stored at -60 °C or below for 12 h.

The collected blood was anticoagulated with EDTA-K2, then temporarily placed in wet ice, and then centrifuged for 10 min at 4 °C and at 2600 g within 2 h. The plasma was temporarily placed in dry ice, and stored in a refrigerator at -60 °C or below within 14 h.

Analytical method: The drug concentrations in plasma and brain tissue were measured by the following LC-MS/MS method.
Instrument: Shimadzu LC-MS/MS (Model: LCMS-8060);
Chromatographic column: GL Sciences Inc., InertSustain, C18, 3 µm, 2.1 × 50 mm;
Ion detection: positive ion mode, m/z 453.10/338.05;
Mobile phase A: aqueous solution containing 0.1% TFA; mobile phase B: methanol solution containing 0.1% TFA;
Flow rate: 0.4 mL min⁻¹

| Gradient: time (min) | mobile phase B (%) |
|---|---|
| 0.50 | 60 |
| 0.60 | 85 |
| 1.80 | 95 |
| 2.50 | 95 |
| 2.60 | 60 |
| 3.00 | stop |

**Pharmacokinetic parameter evaluation index:** pharmacokinetic parameters were calculated in a non-compartmental model using WinNonlin 6.4.

### Results and conclusions:

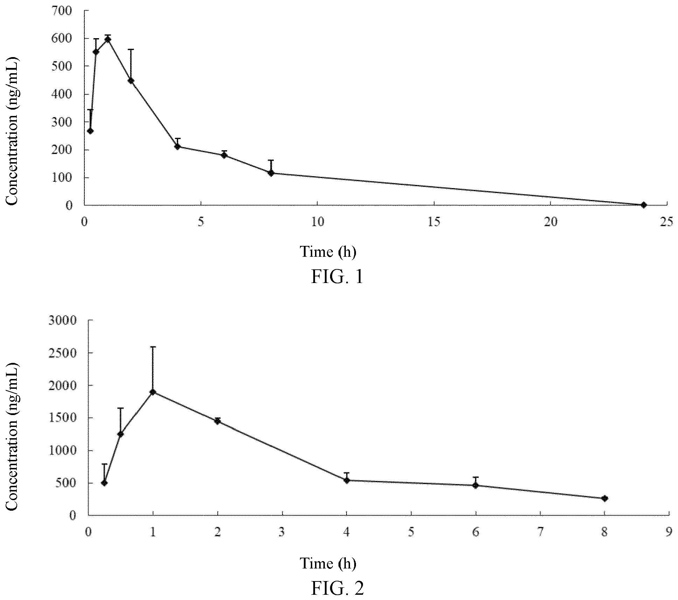

FIG. 1 shows the mean plasma concentration-time curves for mice after administration in Example 5, with pharmacokinetic parameters (PKs) thereof summarized in Table 4 below:

**Table 4: Pharmacokinetic parameters of plasma**

| **Pharmacokinetic parameters** | **Unit** | |
|---|---|---|
| Kel | h-1 | 0.247 |
| t_{1/2} | h | 2.80 |
| Tₘₐₓ | h | 1.00 |
| Cₘₐₓ | ng·mL⁻¹ | 597 |
| AUC₀₋ₜ | ng·h·mL⁻¹ | 3248 |
| AUC_{0-∞} | ng·h·mL⁻¹ | 3257 |
| MRT₀₋ₜ | h | 4.50 |
| MRT_{0-∞} | h | 4.56 |
| Vz/F | mL/kg | 37276 |
| CL/F | mL/h/kg | 9212 |

FIG. 2 shows the drug concentration-time curves for the brain tissue of mice after administration in Example 5, with pharmacokinetic parameters thereof summarized in Table 5 below:

**Table 5: Pharmacokinetic parameters of brain tissue**

| **Pharmacokinetic parameters** | **Unit** | |
|---|---|---|
| Tₘₐₓ | h | 1.00 |
| Cₘₐₓ | ng.g⁻¹ | 1898 |
| AUC₀₋ₜ | ng·h·g⁻¹ | 6457 |
| AUC_{0-∞} | ng·h·g⁻¹ | 7409 |

The experimental results show that the compound has excellent blood-brain barrier permeability. The blood-brain barrier permeability C_{brain}/C_{blood} at 1 h can reach about 3.2, and AUC_{brain}/AUCₚₗₐₛₘₐ can reach about 2.0. Therefore, the compounds of the examples of the present disclosure can more rapidly and effectively permeate the blood-brain barrier to reach the treatment site so as to exert the efficacy.

The above examples illustrate the embodiments of the present disclosure. However, the protection scope of the present application is not limited to the examplary embodiments described above. Any modification, equivalent replacement, improvement, and the like made by those skilled in the art without departing from the spirit and principle of the present disclosure shall fall within the protection scope of the claims of the present application.

## Claims

1. A compound represented by formula (I), or a racemate, a stereoisomer, a tautomer, an isotopically labeled compound, an N-oxide, a hydrate, a solvate, a polymorph, a metabolite, a pharmaceutically acceptable salt, a pharmaceutically acceptable ester or a prodrug compound thereof: 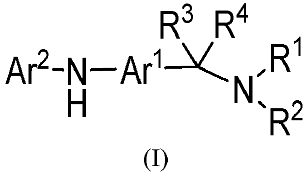 wherein,
R¹ and R², together with the atom attached thereto, form 3- to 20-membered heterocyclyl unsubstituted or optionally substituted with 1, 2 or more R^{b};
R³ and R⁴ are identical or different, and are each independently selected from H, CN, and the following groups unsubstituted or optionally substituted with 1, 2 or more R^{c}: C₁₋₄₀ alkyl, C₃₋₄₀ cycloalkyl, C₆₋₂₀ aryl, 5- to 20-membered heteroaryl, 3- to 20-membered heterocyclyl, C₁₋₄₀ alkyloxy, C₃₋₄₀ cycloalkyloxy, C₆₋₂₀ aryloxy, 5- to 20-membered heteroaryloxy, and 3- to 20-membered heterocyclyloxy; provided that R³ and R⁴ are not both H;
or, R³ and R⁴, together with the atom attached thereto, form the following group unsubstituted or optionally substituted with 1, 2 or more R^{d}: C₃₋₄₀ cycloalkyl or 3- to 20-membered heterocyclyl;
Ar¹ is selected from C₆₋₂₀ aryl and 5- to 20-membered heteroaryl substituted with 1, 2 or more Re;
Ar² is selected from C₆₋₂₀ aryl and 5- to 20-membered heteroaryl substituted with 1, 2 or more R^{f};
each of R^{b}, R^{c}, R^{d}, R^{e} and R^{f} is identical or different, and is independently selected from H, halogen, OH, CN, NO₂, oxo (=O), thio (=S), and the following groups unsubstituted or optionally substituted with 1, 2 or more R^{g}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₁₋₄₀ alkylthio, C₂₋₄₀ alkenylthio, C₂₋₄₀ alkynylthio, C₃₋₄₀ cycloalkylthio, C₃₋₄₀ cycloalkenylthio, C₃₋₄₀ cycloalkynylthio, C₆₋₂₀ arylthio, 5- to 20-membered heteroarylthio, 3- to 20-membered heterocyclylthio, NH₂, -C(O)R¹¹, -C(O)OR¹², -OC(O)R¹³, -S(O)₂R¹⁴, -S(O)₂OR¹⁵, and -OS(O)₂R¹⁶,
each R^{g} is identical or different, and is independently selected from H, halogen, OH, CN, NO₂, oxo (=O), thio (=S), C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, 3- to 20-membered heterocyclyloxy, C₁₋₄₀ alkylthio, C₂₋₄₀ alkenylthio, C₂₋₄₀ alkynylthio, C₃₋₄₀ cycloalkylthio, C₃₋₄₀ cycloalkenylthio, C₃₋₄₀ cycloalkynylthio, C₆₋₂₀ arylthio, 5- to 20-membered heteroarylthio, 3- to 20-membered heterocyclylthio, NH₂, -C(O)C₁₋₄₀ alkyl, -C(O)NH₂, -C(O)NHC₁₋₄₀ alkyl, -C(O)-NH-OH, -COOC₁₋₄₀ alkyl, -COOH, -OC(O)C₁₋₄₀ alkyl, -OC(O)H, -S(O)₂C₁₋₄₀ alkyl, S(O)₂H, -S(O)₂OC₁₋₄₀ alkyl, and -OS(O)₂C₁₋₄₀ alkyl;
R¹¹, R¹², R¹³, R¹⁴, R¹⁵ and R¹⁶ are identical or different, and are each independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, and NH₂.

2. The compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester or the prodrug compound thereof according to claim 1, wherein R¹ and R², together with the atom attached thereto, form 3- to 10-membered heterocyclyl unsubstituted or optionally substituted with 1, 2 or more R^{b};
preferably, R³ and R⁴ are identical or different, and are each independently selected from H, CN, and the following groups unsubstituted or optionally substituted with 1, 2 or more R^{c}: C₁₋₆ alkyl, C₃₋₈ cycloalkyl, C₁₋₆ alkyloxy, and C₃₋₈ cycloalkyloxy; provided that R³ and R⁴ are not both H;
or, R³ and R⁴, together with the atom attached thereto, form the following group unsubstituted or optionally substituted with 1, 2 or more R^{d}: C₃₋₈ cycloalkyl or 3- to 8-membered heterocyclyl;
preferably, Ar¹ is selected from C₆₋₁₀ aryl and 5- to 10-membered heteroaryl substituted with 1, 2 or more R^{e};
preferably, Ar² is selected from C₆₋₁₀ aryl and 5- to 10-membered heteroaryl substituted with 1, 2 or more R^{f};
preferably, R¹ and R², together with the atom attached thereto, form the following group unsubstituted or optionally substituted with 1, 2 or more R^{b}: 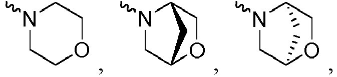 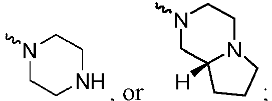
preferably, R³ and R⁴ are identical or different, and are each independently selected from H, C₁₋₆ alkyl, and C₁₋₆ haloalkyl, such as H, methyl, and trifluoromethyl; provided that R³ and R⁴ are not both H;
or, R³ and R⁴, together with the atom attached thereto, form C₃₋₈ cycloalkyl, such as cyclopropyl;
preferably Ar¹ is selected from phenyl substituted with 1, 2 or more R^{e};
preferably, Ar² is selected from the following group substituted with 1 2 or more R^{f}:

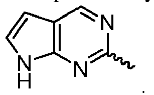

3. The compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester or the prodrug compound thereof according to claim 1 or 2, wherein the compound represented by formula (I) can have a structure represented by formula (II): 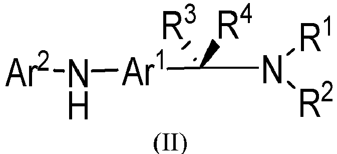 wherein R¹, R², R³, R⁴, Ar¹ and Ar² are independently defined as in claim 1 or 2.

4. The compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester or the prodrug compound thereof according to any one of claims 1 to 3, wherein the compound represented by formula (I) can be selected from the following compounds:

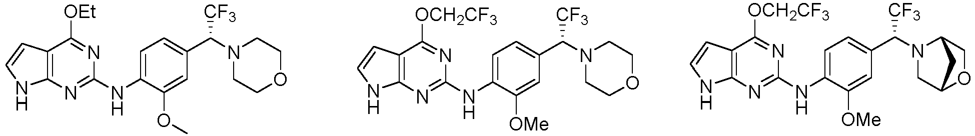

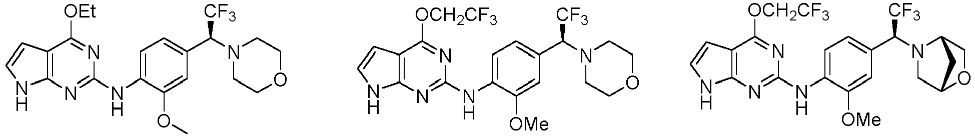

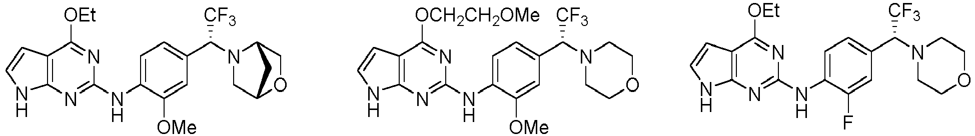

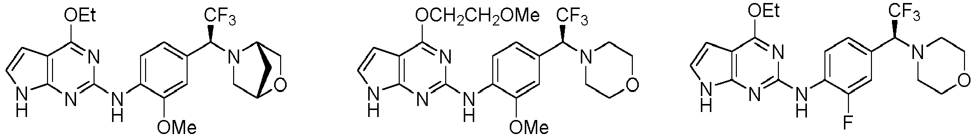

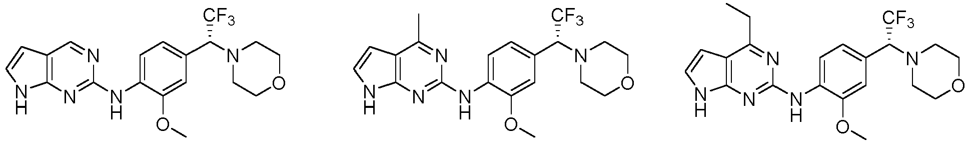

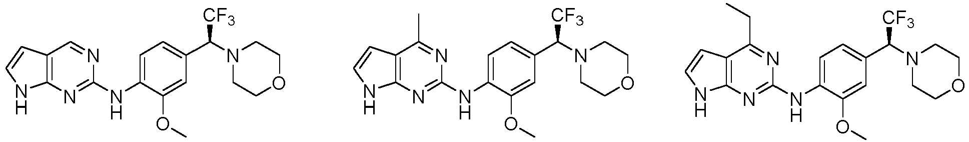

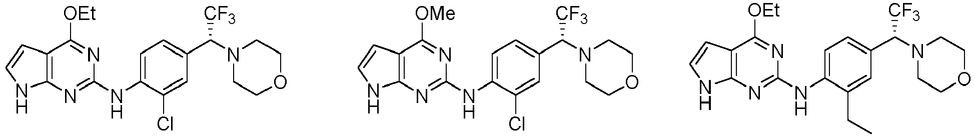

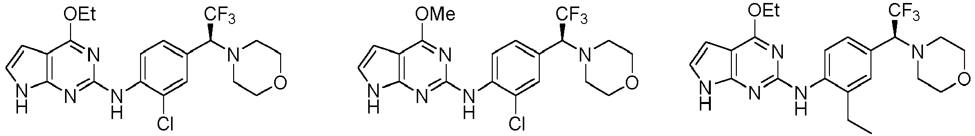

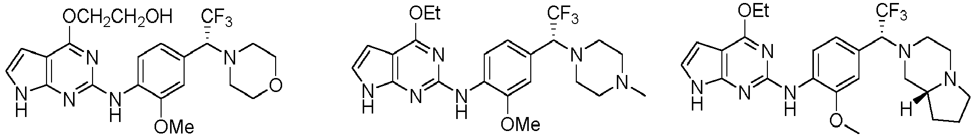

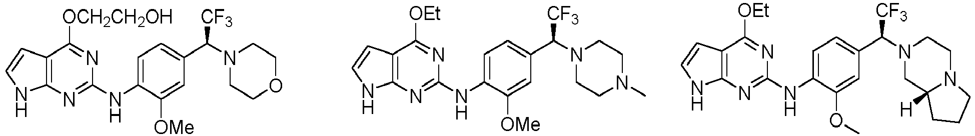

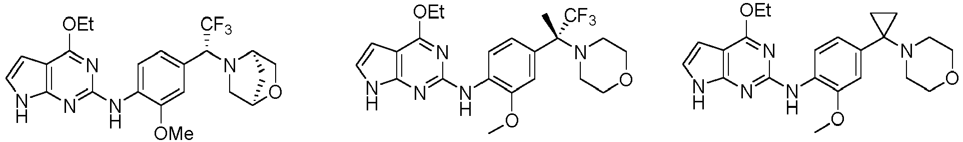

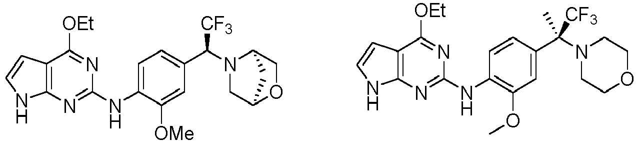

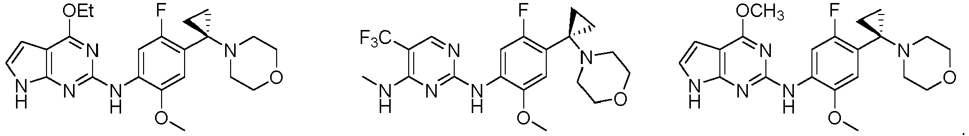

5. A preparation method for the compound, or the racemate, the stereoisomer, the tautomer, the isotopically labeled compound, the N-oxide, the hydrate, the solvate, the polymorph, the metabolite, the pharmaceutically acceptable salt, the pharmaceutically acceptable ester or the prodrug compound thereof according to any one of claims 1 to 4, 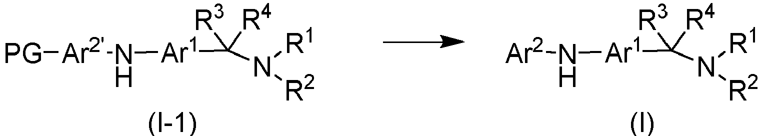
wherein PG, R¹, R², R³, R⁴, Ar¹, Ar² and Ar^{2'} are independently defined as in any one of claims 1 to 4;
preferably, PG can be selected from amino protective groups; wherein, suitable PG can be selected from C₁₋₄₀ alkyl, C₆₋₂₀ aryl C₁₋₄₀ alkyl, C₁₋₄₀ alkylsulfonyl, and C₁₋₄₀ alkylbenzenesulfonyl, such as 4-tosyl, tert-butyl, isopropyl, benzyl, tert-butoxycarbonyl (Boc), 2-biphenyl-2-propoxycarbonyl, benzyloxycarbonyl, fluorenylmethoxycarbonyl (Fmoc), and trifluoroacetyl;
preferably, a compound of formula (I-1) is reacted after the protective group PG is removed off to obtain the compound of formula (I).

6. A compound represented by formula (I-1): 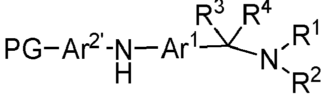 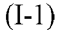 wherein PG is a protective group; Ar^{2'} is a substructure formed with an Ar² group losing one H; R¹, R², R³, R⁴, Ar¹ and Ar² are independently defined as in any one of claims 1 to 4.

7. A preparation method for the compound according to claim 6, comprising reacting a compound of formula (I-2) with a compound of formula (I-3) to obtain the compound represented by formula (I-1); 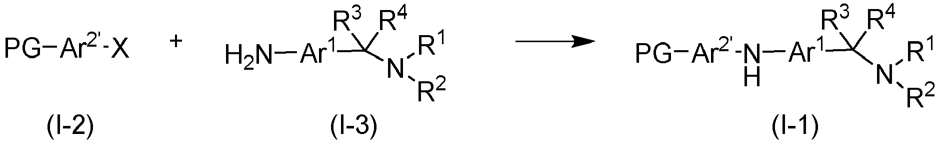
wherein PG, R¹, R², R³, R⁴, Ar¹, Ar² and Ar^{2'} are independently defined as in any one of claims 1 to 4;
preferably, the preparation method can be performed in the presence of a solvent such as an organic solvent; for example, the organic solvent can be selected from at least one of the following: alcohols such as methanol, ethanol, isopropanol, and n-butanol; ethers such as ethyl propyl ether, *n-*butyl ether, anisole, phenetole, cyclohexylmethyl ether, dimethyl ether, diethyl ether, dimethyl glycol, diphenyl ether, dipropyl ether, diisopropyl ether, di-n-butyl ether, diisobutyl ether, diisoamyl ether, dimethoxyethane, isopropyl ethyl ether, methyl tert-butyl ether, tetrahydrofuran, methyltetrahydrofuran, dioxane, dichlorodiethyl ether, and polyethers of ethylene oxide and/or propylene oxide; aliphatic, cycloaliphatic or aromatic hydrocarbons such as pentane, hexane, heptane, octane, nonane, and those that can be substituted with fluorine and chlorine atoms, such as methylene chloride, dichloromethane, trichloromethane, tetrachloromethane, fluorobenzene, chlorobenzene or dichlorobenzene; cyclohexane, methylcyclohexane, petroleum ether, octane, benzene, toluene, chlorobenzene, bromobenzene, and xylene; and esters such as methyl acetate, ethyl acetate, butyl acetate, isobutyl acetate and dimethyl carbonate, dibutyl carbonate or ethylene carbonate;
preferably, the preparation method can be performed in the presence of a catalyst, wherein the catalyst can be a Pd catalyst, and the Pd catalyst can be selected from at least one of Pd₂(dba)₃, Pd(dba)₂, Pd(OAc)₂, Pd[(PPh)₃]₄, Pd[(PPh)₃]₂Cl₂, and Pd₂(dppf)Cl₂; the preparation method can further comprise adding a ligand, wherein the ligand can be an organic phosphine ligand, and the organic phosphine ligand can be selected from at least one of 2-dicyclohexylphosphino-2',6'-diisopropoxybiphenyl (RuPhos), 2-dicyclohexylphosphino-2'-(*N,N*-dimethylamine)-biphenyl (DavePhos), 2,2'-bis-diphenylphosphinoalkyl-[1,1']binaphthyl (BINAP) in a racemic or R- or S-configuration, 1,1-bis(diphenylphosphino)ferrocene (DPPF), and bis-(2-diphenylphosphinophenyl)ether (DPEPhos);
preferably, the preparation method can be performed under the action of a base, wherein the base can be an inorganic base selected from at least one of sodium carbonate, potassium carbonate, potassium bicarbonate, sodium bicarbonate, and cesium carbonate.

8. A pharmaceutical composition comprising a therapeutically effective amount of at least one of the compound, and the racemate, the stereoisomer, the tautomer, the isotopically labeled compound, the N-oxide, the hydrate, the solvate, the polymorph, the metabolite, the pharmaceutically acceptable salt, the pharmaceutically acceptable ester and the prodrug compound thereof according to any one of claims 1 to 4.

9. A method for treating or preventing a disease or disorder associated with LRRK kinase activity, comprising administering to a patient at least one of the compound, and the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, and the prodrug compound thereof according to any one of claims 1 to 4;
preferably, LRRK kinase is preferably LRRK2 kinase, or a mutant or an isoform thereof, or a combination thereof;
preferably, the disease or disorder associated with LRRK kinase, particularly LRRK2 kinase activity, comprises: neurodegenerative diseases, proliferative diseases, diseases associated with protein kinases, lysosomal diseases, Tau diseases, and diseases caused by decreased dopamine levels, such as cancer (e.g., breast cancer), Parkinson's disease, Parkinson's disease (PD) associated with GBA mutations, other α-synucleinopathies, tau protein disease, cognitive impairment or cognitive disorder, Alzheimer's disease, Gaucher disease, Niemann-Pick type C (NPC), argyrophilic grain disease, Pick's disease, corticobasal degeneration, progressive supranuclear palsy, dementia, frontotemporal dementia with parkinsonism linked to chromosome 17 (FTDP-17), craniocerebral injury, stroke, epilepsy, withdrawal symptoms/relapse associated with drug addiction, inflammatory bowel diseases, leprosy, immune system diseases, and the like; preferably, the disease and disorder comprise those associated with the brain, in particular with blood supply to the brain or nerves, such as cognitive impairment or cognitive disorder, Alzheimer's disease, dementia, frontotemporal dementia with parkinsonism linked to chromosome 17 (FTDP-17), craniocerebral injury, stroke, and epilepsy.

10. Use of at least one of the compound, and the racemate, the stereoisomer, the tautomer, the isotopically labeled compound, the N-oxide, the hydrate, the solvate, the polymorph, the metabolite, the pharmaceutically acceptable salt, the pharmaceutically acceptable ester, and the prodrug compound thereof according to any one of claims 1 to 4 for the manufacturing of a medicament;
preferably, the medicament is used for the treatment or prevention of a disease or disorder associated with LRRK kinase activity;
preferably, LRRK kinase is preferably LRRK2 kinase, or a mutant or an isoform thereof, or a combination thereof;
preferably, the disease or disorder associated with LRRK kinase, particularly LRRK2 kinase activity, comprises: neurodegenerative diseases, proliferative diseases, diseases associated with protein kinases, lysosomal diseases, Tau disease, and diseases caused by decreased dopamine levels, such as cancer (e.g., breast cancer), Parkinson's disease, Parkinson's disease (PD) associated with GBA mutations, other α-synucleinopathies, tau protein disease, cognitive impairment or cognitive disorder, Alzheimer's disease, Gaucher disease, Niemann-Pick type C (NPC), argyrophilic grain disease, Pick's disease, corticobasal degeneration, progressive supranuclear palsy, dementia, frontotemporal dementia with parkinsonism linked to chromosome 17 (FTDP-17), craniocerebral injury, stroke, epilepsy, withdrawal symptoms/relapse associated with drug addiction, inflammatory bowel diseases (e.g., Crohn's disease, and ulcerative colitis), leprosy, immune system diseases, and the like;
preferably, the disease and disorder comprise those associated with the brain, in particular with blood supply to the brain or nerves, such as cognitive impairment or cognitive disorder, Alzheimer's disease, dementia, frontotemporal dementia with parkinsonism linked to chromosome 17 (FTDP-17), craniocerebral injury, stroke, and epilepsy.
